(19) Europäisches Patentamt / European Patent Office / Office européen des brevets

(11) EP 2 279 172 B1

(12) EUROPÄISCHE PATENTSCHRIFT

(45) Veröffentlichungstag und Bekanntmachung des Hinweises auf die Patenterteilung:
**26.06.2013 Patentblatt 2013/26**

(21) Anmeldenummer: **09765486.7**

(22) Anmeldetag: **15.05.2009**

(51) Int Cl.:
***C12P 7/04*** *(2006.01)* ***C12P 7/06*** *(2006.01)*
***C12P 7/16*** *(2006.01)* ***C07D 213/20*** *(2006.01)*
***C07D 233/58*** *(2006.01)* ***C07F 5/02*** *(2006.01)*
***C07D 295/037*** *(2006.01)* ***C07C 211/63*** *(2006.01)*
***B01D 11/04*** *(2006.01)* ***C07F 9/54*** *(2006.01)*

(86) Internationale Anmeldenummer:
**PCT/EP2009/003485**

(87) Internationale Veröffentlichungsnummer:
**WO 2009/152906 (23.12.2009 Gazette 2009/52)**

(54) **VERWENDUNG VON IONISCHEN FLÜSSIGKEITEN MIT TETRACYANOBORATANIONEN ALS LÖSUNGSMITTEL ZUR EXTRAKTION VON ALKOHOLEN AUS WÄSSRIGEN LÖSUNGEN**

USE OF IONIC LIQUIDS WITH TETRACYANOBORATE ANIONS AS A SOLVENT FOR EXTRACTION OF ALCOHOLS FROM AQUEOUS SOLUTIONS

UTILISATION DE LIQUIDES IONIQUES COMPRENANT DES ANIONS TÉTRACYANOBORATE EN TANT QUE SOLVANTS POUR L'EXTRACTION D'ALCOOLS À PARTIR DE SOLUTIONS AQUEUSES

(84) Benannte Vertragsstaaten:
**AT BE BG CH CY CZ DE DK EE ES FI FR GB GR HR HU IE IS IT LI LT LU LV MC MK MT NL NO PL PT RO SE SI SK TR**

(30) Priorität: **26.05.2008 EP 08009552**
**09.09.2008 EP 08015819**

(43) Veröffentlichungstag der Anmeldung:
**02.02.2011 Patentblatt 2011/05**

(73) Patentinhaber: **Merck Patent GmbH**
**64293 Darmstadt (DE)**

(72) Erfinder:
- **PITNER, William-Robert**
  **60599 Frankfurt (DE)**
- **SCHULTE, Michael**
  **65474 Bischofsheim (DE)**
- **GÓRAK, Andrzej**
  **58453 Witten (DE)**
- **SANTANGELO, Francesca**
  **44147 Dortmund (DE)**
- **WENTINK, Annebart, Engbert**
  **68161 Mannheim (DE)**

(56) Entgegenhaltungen:
**WO-A2-2010/000357**

- **PAVEL IZÃ K ET AL: "Increased productivity of Clostridium acetobutylicum fermentation of acetone, butanol, and ethanol by pervaporation through supported ionic liquid membrane" APPLIED MICROBIOLOGY AND BIOTECHNOLOGY, SPRINGER, BERLIN, DE, Bd. 78, Nr. 4, 29. Januar 2008 (2008-01-29), Seiten 597-602, XP019586331 ISSN: 1432-0614**
- **CHAPEAUX: "Liquid Phase Behavior of Ionic Liquids with Water and 1-Octanol and Modelingof 1-Octanol/Water Partition Coefficients" J. CHEM. ENG. DATA, Bd. 52, 2007, Seiten 2462-2467, XP002568609**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



Printed by Jouve, 75001 PARIS (FR)

## Beschreibung

Gegenstand der Erfindung ist ein Verfahren zur Flüssig-Flüssig-Extraktion von Alkoholen aus wässrigen Lösungen unter Verwendung mindestens einer Ionischen Flüssigkeit enthaltend ein Tetracyanoboratanion als Lösungsmittel nach Anspruch 1.

Die Extraktion von Alkoholen aus wässrigen Lösungen gewinnt mehr und mehr an Bedeutung durch intensive Diskussion über "weiße Biotechnologie" bzw. dem Einsatz von Alkoholen als Zusatzstoffe zu konventionellen Treibstoffen. Im Rahmen der intensiven Diskussion von Biotreibstoffen, insbesondere von Bioethanol, zeigt sich, dass Biobutanol, d.h. Butanol welches durch Biomasse hergestellt wird, ebenfalls als potentieller Biotreibstoff von Interesse ist.

Biobutanol hat eine Vielzahl von Vorteilen gegenüber anderen Biotreibstoffen, insbesondere

- kann er aufgrund des geringen Dampfdruckes (5,6 hPa gegenüber 58,5 hPa bei Ethanol) und des geringeren Flammpunktes (36°C gegenüber 12°C für Ethanol) leicht mit konventionellen Treibstoffen gemischt werden,
- zeigt deulich geringeres hygroskopisches Verhalten,
- ist der Energiegehalt dem konventioneller Treibstoffe ähnlicher
- ist beispielsweise weniger korrosiv,
- kann fossile Brennstoffe zu 100% ersetzen ohne notwendige Motormodifizierungen und
- kann mit Biodiesel/Diesel gemischt und in Dieselmotoren genutzt werden.

Die Herstellung von Alkoholen durch Biomasse ist seit langem bekannt. Eine Fermentation, die schon in der ersten Hälfte des zwanzigsten Jahrhunderts industriell eingesetzt wurde, ist die ABE-Fermentation (Aceton-Butanol-Ethanol-Fermentation). Damals wurde der Mikroorganismus *Clostridium acetobutyricum* eingesetzt. Derzeit werden für die Butanolherstellung aus Biomasse weitere Bakterien der Gattung *Clostridium,* wie z.B. *C.beijerinckii, C. saccharoperbutylacetonicum und C.tetanomorphum* eingesetzt. Darüber hinaus werden neue Mikroorganismen, insbesondere Bakterien und Hefen, entwickelt, die einen höheren Butanol-Anteil herstellen und zudem auch toleranter gegenüber Butanol in der Fermentationslösung sind. Die Toleranzgrenze liegt jedoch schon bei 2 Gewichtsprozent Butanol bezogen auf die wässrige Lösung.

Die Extraktion von Alkoholen, insbesondere von Butanol, aus einer wässrigen Lösung, und in einer besonderen Anwendungsform die Aufreinigung von Fermentationsbrühen ist daher weiterhin eine Herausforderung. Destillatives Entfernen der Alkohole aus wässrigen Lösungen ist kostenintensiv. Aufgrund des höheren Siedepunktes von Butanol im Vergleich zu Wasser ist die Rektifikation als Trennverfahren für Butanol nicht wirtschaftlich durchführbar. Bisherig genutzte Extraktionsverfahren benötigen Extraktionsmedien, die in der Regel entflammbar, umweltschädlich oder toxisch sind.

Für die Extraktion von Alkoholen aus Fermentationsbrühen wird zur Zeit am häufigsten der Fettalkohol Oleylalkohol untersucht. Dieser hat jedoch den Nachteil, ein Emulgator zu sein, der bei Verwendung in der Flüssig-Flüssig-Extraktion zu Schäumen führen kann. Eine Schaumphasenbildung führt bei Extraktionskolonnen zu einem höheren Druckverlust und erschwert zudem die Phasentrennung der beiden flüssigen Phasen. Oleylalkohol wird zudem als "reizend für Atemwege" eingestuft.

Es besteht daher ein Bedarf an neuen Extraktionsmedien für die Extraktion von Alkoholen aus wässrigen Lösungen, die alternativ zu den herkömmlichen Verbindungen eingesetzt werden können und die einen hohen Verteilungskoeffizienten und eine hohe Selektivität für den zu extrahierenden Alkohol haben. Die Anforderungen and die neuen Medien sind daher:

- gute Selektivität für die Aufnahme des zu extrahierenden Alkohols, idealerweise mit einem Selektivitätskoeffizient > 100,
- guter Verteilungskoeffizient, beispielsweise mit einem Verteilungskoeffizient $D_{Alkohol} > 2$),
- nicht mischbar mit Wasser,
- geringe Wasseraufnahme von der wässrigen Lösung,
- nicht toxisch für den Mikroorganismus, falls die wässrige Lösung eine Fermentationsbrühe ist.

Aufgabe der vorliegenden Erfindung ist demgemäß die Bereitstellung neuer Extraktionsmedien für die Flüssig-Flüssig-Extraktion von Alkoholen aus wässrigen Lösungen.

Diese Aufgabe wird erfindungsgemäß durch die Merkmale des Hauptanspruchs und der nebengeordneten Ansprüche gelöst.

Überraschend wurde gefunden, dass sich Ionische Flüssigkeiten enthaltend Tetracyanoboratanionen in besonderem Maße als Lösungsmittel für eine deartige Flüssig-Flüssig-Extraktion von Alkoholen aus wässrigen Lösungen eignen.

Gegenstand der Erfindung ist daher ein Verfahren zur Flüssig-Flüssig-Extraktion von Alkoholen aus wässrigen

Lösungen unter Verwendung mindestens einer Ionischen Flüssigkeit enthaltend Tetracyanoboratanionen als Lösungsmittel.

Idealerweise hat die Ionische Flüssigkeit enthaltend

Tetracyanoboratanionen die Eigenschaft, mit der wässrigen Lösung enthaltend mindestens einen Alkohol eine zweiphasige Mischung auszubilden.

Die Ionischen Flüssigkeiten mit Tetracyanoboratanionen sowie deren Herstellung und Verwendungen als Lösungsmittel für viele synthetische oder katalytische Reaktionen, z.B. Friedel-Crafts-Acylierung und - Alkylierung, Diels-Alder-Cycloadditionen, Hydrogenierungs- und Oxidationsreaktionen, Heck-Reaktionen, als Vorstufen für die Herstellung von Flüssigkristallverbindungen und von Wirkstoffen, u.a. für Arznei- und Pflanzenschutzmittel oder als nichtwässriger Elektrolyt gegebenenfall in Kombination mit anderen bekannten Elektrolyten oder als Phasentransferkatalysator oder als Medium zur Heterogenisierung von homogenen Katalysatoren sind beispielsweise aus WO 2004/072089 bekannt.

Der Einsatz von Tetrapropylammoniumtetracyanoborat als Bestandteil einer Mehrphasenmembran für den Einsatz in einem technischen Membranverfahren zur Reinigung von Flüssigkeitsgemischen, der sogenannten Pervaporation, ist aus DE 10 2006 024 397 bekannt. Beschriebene Flüssigkeitsgemische sind Kohlenhydrate oder Alkohole in wässrigen Lösungen. Die im Membranmaterial ablaufenden physikalischen Vorgänge können mit dem Lösungs-Diffusions-Modell beschrieben werden. Nach der Modellvorstellung dringt eine Komponente des zu trennenden Gemischs bevorzugt in die Membran ein und wird an deren innerer Oberfläche adsorbiert bzw im gesamten Membranmaterial gelöst. Zur Aussenseite der Membran hin wird die Konzentration der gut löslichen Komponente geringer, da sie an der Membranoberfläche verdampft und somit kontinuierlich abgezogen wird. Über dem Membranquerschnitt stellt sich so ein Konzentrationsgefälle ein, welches als treibende Kraft für die Diffusion des Permeats durch die Membran gilt.

Im Gegensatz zu dem beschriebenen Pervaporationsverfahren handelt es sich bei dem erfindungsgemäßen Verfahren um eine Flüssig-Flüssig-Extraktion. Bei der Flüssig-Flüssig-Extraktion, bei der zwei Flüssigkeitsphasen beteiligt sind, wird der Wertstoff, in diesem Fall der Alkohol, aus der Trägerphase in die Extraktphase überführt. Zwischen beiden Phasen stellt sich ein Phasengleichgewicht bezüglich der Wertstoffkonzentration nach dem Nernst'schen Verteilungsgesetz ein:

$$D_{Alkohol} = \frac{C^{IL}_{Alkohol}}{C^{AQ}_{Alkohol}}$$

$D_{Alkohol}$ = Nernst'scher Verteilungskoeffizient für den Wertstoff, hier Alkohol $C^{IL}_{Alkohol}$: Konzentration des Wertstoffes, hier Alkohol, in der Ionischen Flüssigkeit

$C^{AQ}_{Alkohol}$: Konzentration des Wertstoffes, hier Alkohol, in der wässrigen Phase

Die Selektivität S wird definiert als Quotient der Nernst'schen Verteilungskoeffizienten von Alkohol zu Wasser:

$$S = \frac{D_{Alkohol}}{D_{Wasser}}$$

Bei der Flüssig-Flüssig-Extraktion handelt es sich um ein Trennverfahren, in dem der Stofftransport zwischen zwei flüssigen Phasen stattfindet und durch das sich einstellende thermodynamische Gleichgewicht laut dem Nernstschen Verteilungskoeffizienten limitiert wird. Die Pervaporation hingegen wird durch das thermodynamische Gleichgewicht nicht limitiert, weil der Trennmechanismus die unterschiedlichen Transporteigenschaften der Komponenten in der Membran sind. Zudem findet der Stofftransport bei der Flüssig-Flüssig-Extraktion zwischen den zwei flüssigen Phasen statt, wobei bei der Pervaporation zwischen einer flüssigen, einer festen und einer dampfförmigen Phase stattfindet. Die Pervaporation ist im Gegensatz zur Flüssig-Flüssig-Extraktion daher abhängig vom eingestellten Druck auf der Permeatseite.

In der Veröffentlichung von Pavel Izák et al, Appl. Microbiol Biotechnol 2008, 78, 597-602, wird ein Pervaporationsverfahren mit Hilfe einer Polydimethylsiloxan-Ultrafiltrationsmembran beschrieben, wobei die Membran eine Porengröße von 60 nm hat und mit 15 Gewichtsprozent der Ionischen Flüssigkeit Tetrapropylammonium Tetracyanoborat imprägniert ist.

In der Veröffentlichung von A. Chapeaux et al, J. Chem. Eng. Data 2007, 52, 2462-2467, werden die Löslichkeiten von verschiedenen Ionischen Flüssigkeiten, einschließlich von 1-Ethyl-3-methylimidazolium Tetracyanoborat, in Wasser oder in 1-Octanol beschrieben. Die Tabelle 10 dieser Veröffentlichung listet berechnete Verteilungskoeffizienten für die verschiedenen Ionischen Flüssigkeiten, die das Verhältnis der Konzentration der Ionischen Flüssigkeit in 1-Octanol zur Konzentration der Ionischen Flüssigkeit in Wasser beschreiben. Der Wert für 1-Ethyl-3-methylimidazolium Tetracyanoborat ist 0.169. Der Wert für 1-Butyl-3-methylpyridinium Bis(trifluormethansulfonyl)imid ist 1.62. Der Wert für 1-Hexyl-3,5-dimethylpyridinium Bis(trifluormethansulfonyl)imid ist 23.8.

Die erfindungsgemäße Flüssig-Flüssig-Extraktion von Alkoholen aus wässrigen Lösungen unter Verwendung mindestens einer Ionischen Flüssigkeit enthaltend Tetracyanoboratanionen wird, nach Anspruch 1, durchgeführt, indem

a) die wässrige Lösung enthaltend mindestens einen Alkohol bereitgestellt wird,
b) die wässrige Lösung aus a) mit der mindestens einen Ionischen Flüssigkeit enthaltend Tetracyanoboratanionen intensiv gemischt wird, so dass die Ionische Flüssigkeit in der Lage ist, mindestens einen Teil des Alkohols aus der wässrigen Lösung zu extrahieren und mit diesem Alkohol eine mindestens einphasige Mischung herzustellen,
c) abtrennen der mindestens einphasigen Mischung aus b) von der wässrigen Lösung,
d) trennen der einphasigen Mischung aus b) in die Komponenten Alkohol und Ionische Flüssigkeit, und gegebenenfalls
e) zurückführen der Ionischen Flüssigkeit aus d) in Schritt b).

Die erfindungsgemäße Flüssig-Flüssig-Extraktion, wie beschrieben, kann im Batch-Verfahren durchgeführt werden. Sie kann aber auch kontinuierlich oder halbkontinuierlich durchgeführt werden. Dabei kann die Flüssig-Flüssig-Extraktion sowohl in der Gegenstrom-, Gleichstrom als auch in der Kreuzstromfahrweise und in einer oder mehrerer Stufen durchgeführt werden. Die Flüssig-Flüssig-Extraktion kann sowohl in einer einstufigen oder mehrstufigen Mixer-settler-Batterie oder aber auch in einer Extraktionkolonne erfolgen. Das erfindungsgemäße Verfahren kann in allen dem Fachmann bekannten Extrakionsapparaturen und Fahrweisen durchgeführt werden, beispielsweise dokumentiert durch die Fachliteratur von J. Rydberg, M. Cox, C. Muskas, G.R. Chopppin, 2004, Solvent Extraction Principles and Practice oder R.H. Perry, 1984, Perry's chemical engineer's handbook.

Eine Variante einer Flüssig-Flüssig-Extraktion ist in Figur 1 beschrieben. Die Bezeichnungen der Figur 1 ist wie folgt:

[1] Reaktionsgefäß enthaltend die wässrige Lösung enthaltend mindestens einen Alkohol und die erfindungsgemäße Ionische Flüssigkeit, insbesondere ein in-situ Fermenter
[2] Rückgewinnungseinheit
[3] Zugabe der wässrigen Lösung enthaltend mindestens einen Alkohol, insbesondere des Fermentationsmediums inklusive Mikroorganismen und Ionische Flüssigkeit
[4] Wässrige Phase der wässrigen Lösung enthaltend mindestens einen Alkohol, insbesondere bei der Fermentation auch die Mikroorganismen
[5] Phase der Ionischen Flüssigkeit, gegebenenfalls enthaltend den Teil des extrahierten Alkohols bildend die mindestens einphasige Mischung
[6] Strom zur Rückgewinnungseinheit enthaltend die Phase der Ionischen Flüssigkeit, enthaltend den Teil des extrahierten Alkohols
[7] Extrahierter und abgetrennter Alkohol
[8] Gegebenenfalls abgeführter Strom der wässrigen Phase, insbesondere der Fermentationsbrühe
[9] Rückführung der aufgereinigten Ionischen Flüssigkeit.

In [1] wird die wässrige Phase enthaltend den mindestens einen Alkohol, insbesondere die Fermentationsbrühe, enthaltend Fermentationsmedium und Mikroorganismen, mit der ionischen Flüssigkeit in Kontakt gebracht und vermischt. In [1] erfolgt im Falle der bevorzugten Ausführungsform der Fermentation zugleich die Fermentation, d.h. die Produktion des Alkohols durch die Mikroorganismen und Abtrennung des Alkohols aus der Fermentationsbrühe durch Extraktion mittels ionischer Flüssigkeit. Nach trennen der Phasen in [4] und [5] wird die ionische Flüssigkeit mittels Strom [6] in eine Rückgewinnungseinheit [2] geführt und das Butanol abgetrennt, welches im Strom [7] abgeführt wird. Im Strom [9] wird die regenerierte ionische Flüssigkeit in [1] zurückgeführt.

Eine Variante einer erfindungsgemäßen Fermentation mit Hilfe einer Extraktionskolonne ist in Figur 2 beschrieben.

Die Bezeichnung der Figur 2 ist wie folgt:

[1] Fermenter
[2] Rückgewinnungseinheit

[3] Zugabe des Fermentationsmediums inklusive Mikroorganismen
[4] Fermentationsbrühe
[5] Phase der Ionischen Flüssigkeit, gegebenenfalls enthaltend den Teil des extrahierten Alkohols bildend die mindestens einphasige Mischung
[6] Wässrige Phase der wässrigen Lösung enthaltend mindestens einen Alkohol
[7] Abgeführte wässrige Phase, wobei diese in [1] auch zurückgeführt werden kann
[8] Gegebenenfalls abgeführter Strom der wässrigen Phase, Fermentationsbrühe
[9] Extraktionskolonne
[11] Extrahierter und abgetrennter Alkohol
[10] Strom zur Rückgewinnungseinheit enthaltend die Phase der Ionischen Flüssigkeit, enthaltend den Teil des extrahierten Alkohols
[12] Zellrückführung, enthaltend die Mikroorganismen und teilweise Fermentationsmedium
[13] Zellabtrennungseinheit
[14] Wässrige Phase der wässrigen Lösung enthaltend mindestens einen Alkohol
[15] Zugabe der ionischen Flüssigkeit

In [1] wird die Fermentationsbrühe, enthaltend Fermentationsmedium und Mikroorganismen gerührt und begast. Mittels Strom [4] wird ein Teil der Fermentationsbrühe der Zellabtrennungseinheit zugführt. In der Zellabtrennungseinheit [13] werden die Zellen bzw. Mikroorganismen getrennt. Die Zellen bzw. Mikroorganismen werden mittels Strom [12] in den Fermenter [1] zurückgeführt. Die abgetrennte wässrige Lösung enthaltend mindestens einen Alkohol wird mittels Strom [14] in die Extraktionskolonne [9] geführt. In der Extraktionskolonne [9] werden die beiden Phasen, wässrige Phase enthaltend mindestens einen Alkohol und ionische Flüssigkeit gegebenenfalls enthaltend den Teil des extrahierten Alkohols, in Kontakt gebracht. In der Extraktionskolonne wird der Alkohol extrahiert, d.h. ein teil geht von der wässrigen Phase in die ionische Flüssigkeitsphase über. Die wässrige Phase wird halb-kontinuierlich in Strom [7] abgezogen und teilweise oder aber auch ganz in [1] zurückgeführt. Mittels Strom [10] wird die Phase der Ionischen Flüssigkeit, enthaltend den Teil des extrahierten Alkohols in eine Rückgewinnungseinheit geführt. In der Rückgewinnungseinheit [2] wird der Alkohol von der ionischen Flüssigkeit abgetrennt und in Strom [11] abgeführt. Die regenierierte Strom [8] ,die regenerierte ionische Flüssigkeit enthaltend kein Alkohol, wird in die Extraktionskolonne [9] zurückgeführt.

Generell geeignete Alkohole sind sowohl Monohydroxyalkohole, bevorzugt mit 2, 3 oder 4 C-Atomen, als auch Alkohole mit mehr als einer Hydroxygruppe, beispielsweise Diole, bevorzugt mit 3, 4 oder 5 C-Atomen.
Ausgewählte Diole sind beispielsweise 2,3-Butandiol oder 1,3-Propandiol.

Nach Anspruch 1 wird der mindestens eine Alkohol aus der Gruppe Ethanol, Isopropanol, Propanol, n-Butanol oder Isomere des n-Butanol oder Mischungen derselben gewählt. Besonders bevorzugt wird das erfindungsgemäße Verfahren für die Extraktion von n-Butanol, Isomeren des n-Butanol oder Mischungen derselben verwendet. Ganz besonders bevorzugt wird das erfindungsgemäße Verfahren für die Extraktion von n-Butanol verwendet.

Das durch Fermentation hergestellte sogenannte Biobutanol enthält als Hauptkomponente n-Butanol und als Nebenbestandteile isomere Butanole. Der Begriff n-Butanol ist gleichbedeutend mit 1-Butanol. Die Verwendung des Begriffs Butanol ist im folgenden als identisch zu n-Butanol anzusehen.

Die wässrige Lösung des erfindungsgemäßen Verfahrens beinhaltet den Alkohol in einer Konzentration von 0.5 bis10 Gewichtsprozent bezogen auf die wässrige Lösung. Für wässrige Lösungen aus Biomasse, d.h. für Fermentationsbrühen, liegt der Alkohol in einer Konzentration von 0,1 bis 3 Gewichtsprozent vor, bevorzugt in einer Konzentration von 0,5 bis 2 Gewichtsprozent vor, bezogen auf die Fermentationsbrühe. Eine natürliche Grenze ist die Produktionsgrenze des Mikroorganismus. Es ist jedoch auch möglich, die Fermentationsbrühe vorher aufzukonzentrieren und dann das erfindungsgemäße Verfahren durchzuführen.

In einer bevorzugten Ausführungsform der Erfindung ist die wässrige Lösung enthaltend mindestens einen Alkohol eine Fermentationsbrühe, insbesondere eine Fermentationsbrühe aus einer Aceton-Butanol-Ethanol-Fermentation (ABE-Fermentation).

Für die ABE-Fermentation wurde der anfangs verwendete Mikroorganismus *Clostridium beijerinckii* weiterentwickelt zum *Clostridium beijerinckii BA101,* der eine Butanolkonzentration von bis zu 17,8 g/l produzieren bzw. tolerieren kann und zwar mit im Vergleich zu definierten Nährmedien, beispielsweise günstigerem Glukose-Com Steep Liquor-Medium. Die Konzentration des Glukose-Corn Steep Liquor-Medium beträgt 60g/l. Wichtige Nebenprodukte bei dieser Fermentation sind Aceton und Ethanol mit Konzentrationen von 5,5 g/l und 1 g/l. Weitere Nebenprodukte mit sehr geringen Konzentrationen sind Essigsäure und Buttersäure.

Es gehört zum generellen Fachwissen, bei welcher Temperatur die erfindungsgemäße Flüssig-Flüssig-Extraktion durchgeführt wird. Im Falle der besonderen Ausführungsform der Extraktion des Alkohols aus einer in-situ-Fermentation, wie später definiert, ist beispielsweise die ideale Temperatur des Mikroorganismus zu beachten, bei der die Produktion des Alkohols bevorzugt stattfinden kann.

In einer bevorzugten Ausführungsform der vorliegenden Erfindung ist das Kation der Ionischen Flüssigkeit

enthaltend Tetracyanoboratanionen hydrophob.

Geeignete Kationen sind organische Kationen, insbesondere organischen Kationen ausgewählt aus der Gruppe umfassend Ammonium-, Phosphonium-, Sulfonium, Uronium-, Thiouronium-, Guanidiniumkationen oder um heterocyclische Kationen.

Aus der Gruppe der Ammonium-, Phosphonium- oder Sulfonium-Tetracyanoborate sind die Verbindungen der Formeln (1), (2) oder (3) geeignet,

$[NR_4]^+ [B(CN)_4]^-$ (1),

$[PR_4]^+ [B(CN)_4]^-$ (2),

$[SR_3]^+ [B(CN)_4]^-$ (3),

wobei
R jeweils unabhängig voneinander

- ein geradkettiges oder verzweigtes Alkyl mit 1-20 C-Atomen,
- ein geradkettiges oder verzweigtes Alkenyl mit 2-20 C-Atomen und einer oder mehreren Doppelbindungen,
- ein geradkettiges oder verzweigtes Alkinyl mit 2-20 C-Atomen und einer oder mehreren Dreifachbindungen,
- gesättigtes, teilweise oder vollständig ungesättigtes Cycloalkyl mit 5-7 C-Atomen, das mit Alkylgruppen mit 1-6 C-Atomen substituiert sein kann, bedeutet, mit der Bedingung, dass mindestens zwei Substituenten R mindestens 5 C-Atome haben.

Aus der Gruppe der Uronium- oder Thiouronium-Tetracyanoborate sind die Verbindungen der Formeln (4) oder (5) geeignet,

$[C(R^3R^4N)(OR^5)(NR^6R^7)]^+ [B(CN)_4]^-$ (4),

$[C(R^3R^4N)(SR^5)(NR^6R^7)]^+ IB(CN)_4]^-$ (5),

wobei
$R^3$ bis $R^7$ jeweils unabhängig voneinander

- H, wobei H für $R^5$ ausgeschlossen wird,
- ein geradkettiges oder verzweigtes Alkyl mit 1 bis 20 C-Atomen, geradkettiges oder verzweigtes Alkenyl mit 2-20 C-Atomen und einer oder mehreren Doppelbindungen,
- geradkettiges oder verzweigtes Alkinyl mit 2-20 C-Atomen und einer oder mehreren Dreifachbindungen,
- gesättigtes, teilweise oder vollständig ungesättigtes Cycloalkyl mit 3-7 C-Atomen, das mit Alkylgruppen mit 1-6 C-Atomen substituiert sein kann, bedeutet.

Aus der Gruppe der Guanidinium-Tetracyanoborate sind die Verbindungen der Formel (6) geeignet,

$[C(NR^8R^9)(NR^{10}R^{11})(NR^{12}R^{13})]^+ [B(CN)_4]^-$ (6),

wobei
$R^8$ bis $R^{13}$ jeweils unabhängig voneinander

- H,
- ein geradkettiges oder verzweigtes Alkyl mit 1 bis 20 C-Atomen,
- ein geradkettiges oder verzweigtes Alkenyl mit 2-20 C-Atomen und einer oder mehreren Doppelbindungen,
- ein geradkettiges oder verzweigtes Alkinyl mit 2-20 C-Atomen und einer oder mehreren Dreifachbindungen,
- gesättigtes, teilweise oder vollständig ungesättigtes Cycloalkyl mit 3-7 C-Atomen, das mit Alkylgruppen mit 1-6 C-Atomen substituiert sein kann, bedeutet.

Aus der Gruppe der Tetracyanoborate mit heterocyclischem Kation sind die Verbindungen der Formel (7) geeignet,

$[HetN]^+ [B(CN)_4]^-$ (7),

wobei

- HetN$^+$ ein heterocyclisches Kation, ausgewählt aus der Gruppe

Imidazolium

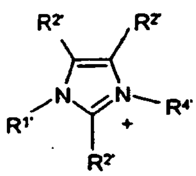

,

1H-Pyrazolium

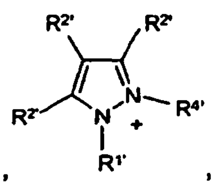

,

3H-Pyrazolium

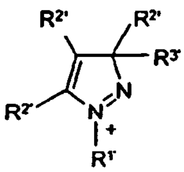

,

4H-Pyrazolium

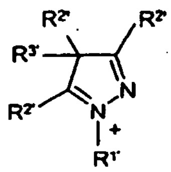

,

1-Pyrazolinium

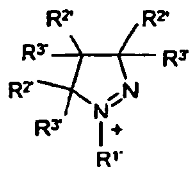

,

2-Pyrazolinium

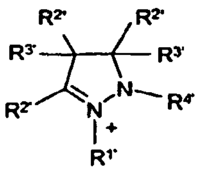

,

3-Pyrazolinium

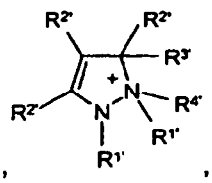

,

2,3-Dihydro-Imidazolinium

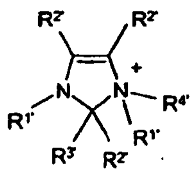

,

4,5-Dihydro-Imidazolinium

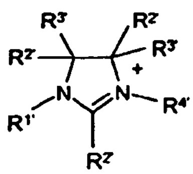

,

2,5-Dihydro-Imidazolinium

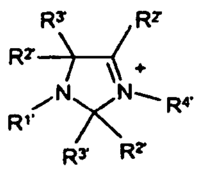

,

Pyrrolidinium

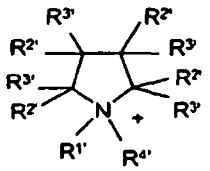

,

1,2,4-Triazolium

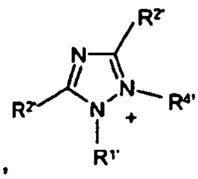

,

1,2,4-Triazolium

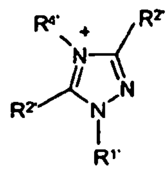

,

1,2,3-Triazolium

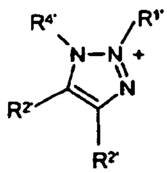

,

1,2,3-Triazolium

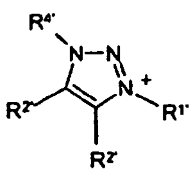

,

Pyridinium

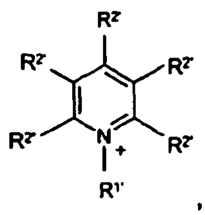

,

Pyridazinium

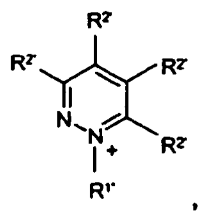

,

Pyrimidinium

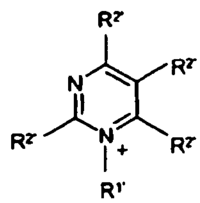

,

Piperidinium

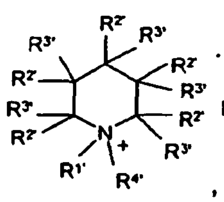

,

Morpholinium

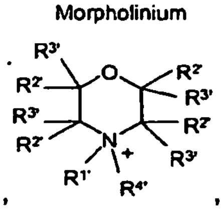

,

Piperazinium

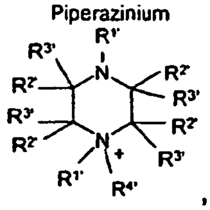

,

Piperazinium

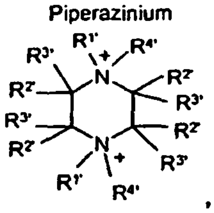

,

Pyrazinium

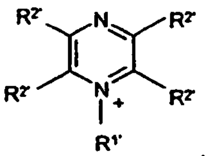

,

Thiazolium

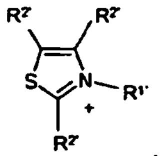

,

Oxazolium

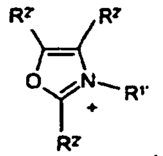

,

Indolium

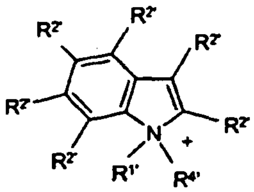

,,

Chinolinium

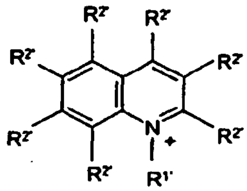

,

Isochinolinium

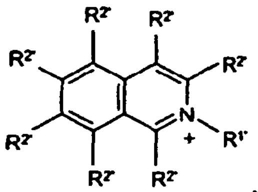

,

Chinoxalinium

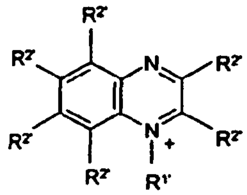

oder

Indolinium

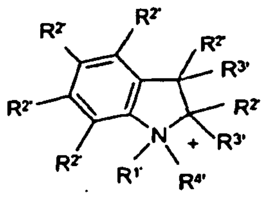

bedeutet, wobei die Substituenten

$R^{1'}$ bis $R^{4'}$ jeweils unabhängig voneinander

- ein geradkettiges oder verzweigtes Alkyl mit 1-20 C-Atomen,
- ein geradkettiges oder verzweigtes Alkenyl mit 2-20 C-Atomen und einer oder mehreren Doppelbindungen,
- ein geradkettiges oder verzweigtes Alkinyl mit 2-20 C-Atomen und einer oder mehreren Dreifachbindungen,
- gesättigtes, teilweise oder vollständig ungesättigtes Cycloalkyl mit 5-7 C-Atomen, das mit Alkylgruppen mit 1-6 C-Atomen substituiert sein kann, bedeutet,

wobei die Substituenten $R^{1'}$, $R^{2'}$, $R^{3'}$ und/oder $R^{4'}$ zusammen auch ein Ringsystem bilden können.

Unter vollständig ungesättigten Substituenten werden im Sinne der vorliegenden Erfindung auch aromatische Substituenten verstanden.

Als Substituenten R und $R^3$ bis $R^{13}$ der Verbindungen der Formeln (1) bis (6) kommen erfindungsgemäß jeweils unabhänig voneinander bevorzugt in Frage $C_6$- bis $C_{18}$-, insbesondere $C_8$- bis $C_{14}$-Alkylgruppen.

Die Substituenten R in den Verbindungen der Formel (1), (2) oder (3) können dabei gleich oder verschieden sein. Für Ammonium- oder Phosphonium-Tetracyanoborate der Formeln (1) oder (2) sind bevorzugt drei Substituenten R gleich und ein Substituent R ist verschieden. Für die Sulfonium-Tetracyanoborate der Formel (3) sind zwei Substituenten R bevorzugt gleich und ein Substituent R ist verschieden.

Die Substituenten R sind insbesondere bevorzugt Pentyl, Hexyl, Octyl, Decyl, Dodecyl oder Tetradecyl.

Bis zu vier Substituenten des Guanidinium-Kations $[C(NR^8R^9)(NR^{10}R^{11})(NR^{12}R^{13})]^+$ können auch paarweise derart verbunden sein, dass mono-, bi- oder polycyclische Kationen entstehen.

Ohne Einschränkung der Allgemeinheit sind Beispiele für solche Guanidinium-Kationen:

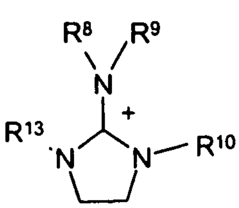 , 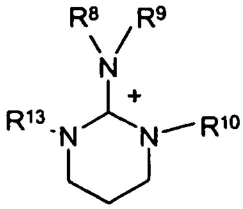 , 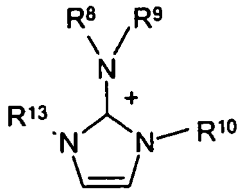 , 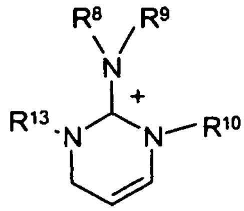 ,

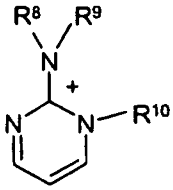 , 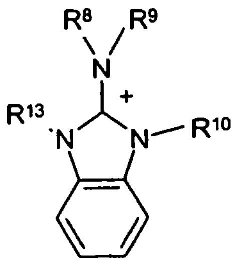 , 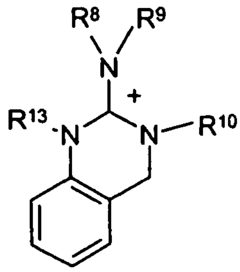 , 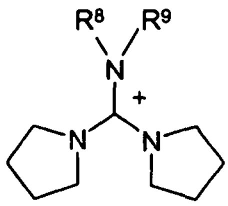

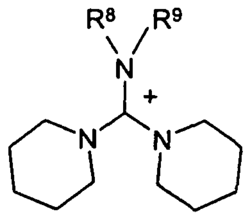 oder 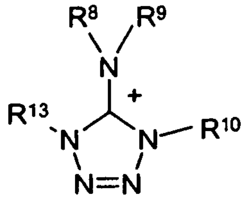

wobei die Substituenten $R^3$ bis $R^{10}$ und $R^{13}$ eine zuvor angegebene oder besonders bevorzugte Bedeutung haben können.

Gegebenenfalls können die Carbocyclen oder Heterocyclen der zuvor angegebenen Guanidinium-Kationen noch durch $C_1$- bis $C_6$-Alkyl substituiert sein.

Bis zu vier Substituenten des Uroniumkations $[C(R^3R^4N)(OR^5)(NR^6R^7)]^+$ oder des Thiouroniumkations $[C(R^3R^4N)(SR^5)(NR^6R^7)]^+$ können auch paarweise derart verbunden sein, dass mono-, bi- oder polycyclische Kationen entstehen.

Ohne Einschränkung der Allgemeinheit sind Beispiele für solche Kationen im Folgenden angegeben, wobei Y = O oder S bedeutet:

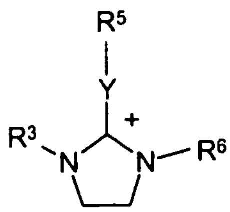 , 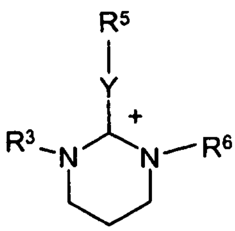 , 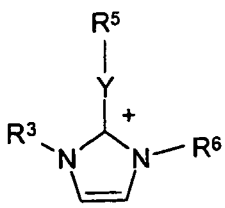 , 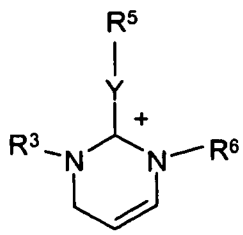 ,

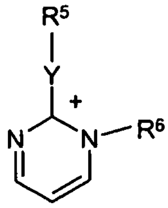,
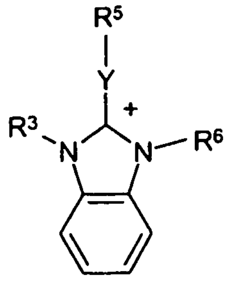,
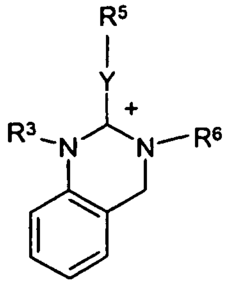,
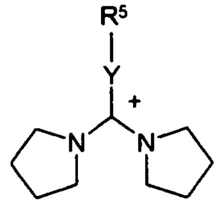

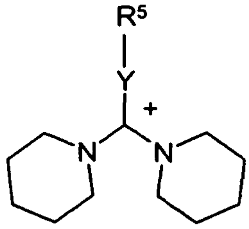 oder 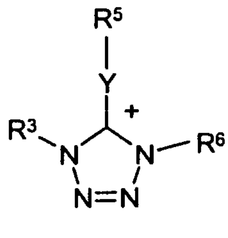,

wobei die Substituenten $R^3$, $R^5$ und $R^6$ eine zuvor angegebene oder besonders bevorzugte Bedeutung haben können. Gegebenenfalls können die Carbocyclen oder Heterocyclen der zuvor angegebenen Kationen noch durch $C_1$- bis $C_6$-Alkyl substituiert sein.

Die Substituenten $R^3$ bis $R^{13}$ sind jeweils unabhängig voneinander bevorzugt eine geradkettige oder verzweigte Alkylgruppe mit 1 bis 16 C-Atomen. Die Substituenten $R^3$ und $R^4$, $R^6$ und $R^7$, $R^8$ und $R^9$, $R^{10}$ und $R^{11}$ und $R^{12}$ und $R^{13}$ in Verbindungen der Formeln (4) bis (6) können dabei gleich oder verschieden sein. Besonders bevorzugt sind $R^3$ bis $R^{13}$ jeweils unabhängig voneinander Methyl, Ethyl, n-Propyl, Isopropyl, n-Butyl, tert.-Butyl, sek.-Butyl, Phenyl, Hexyl oder Cyclohexyl, ganz besonders bevorzugt Methyl, Ethyl, n-Propyl, Isopropyl, n-Butyl oder Hexyl.

Als Substituenten $R^{1'}$ bis $R^{4'}$ von Verbindungen der Formel (7) kommen erfindungsgemäß dabei neben H bevorzugt in Frage: $C_1$- bis $C_{20}$, insbesondere $C_1$- bis $C_{12}$-Alkylgruppen.

Die Substituenten $R^{1'}$ und $R^{4'}$ sind jeweils unabhängig voneinander insbesondere bevorzugt Methyl, Ethyl, Isopropyl, Propyl, Butyl, sek.-Butyl, tert.-Butyl, Pentyl, Hexyl, Octyl, Decyl, Dodecyl, Cyclohexyl, Phenyl oder Benzyl. Sie sind ganz besonders bevorzugt Methyl, Octyl, Decyl oder Dodecyl. In Pyrrolidinium-, Piperidinium- oder Morpholinium-Verbindungen sind die beiden Substituenten $R^{1'}$ und $R^{4'}$ bevorzugt unterschiedlich.

Der Substituent $R^{2'}$ oder $R^{3'}$ ist jeweils unabhängig voneinander insbesondere H, Methyl, Ethyl, Isopropyl, Propyl, Butyl, sek.-Butyl, tert.-Butyl, Cyclohexyl, Phenyl oder Benzyl. Besonders bevorzugt ist $R^{2'}$ H, Methyl, Ethyl, Isopropyl, Propyl, Butyl oder sek.-Butyl. Ganz besonders bevorzugt sind $R^{2'}$ und $R^{3'}$ H.

Die $C_1$-$C_{12}$-Alkylgruppe ist beispielsweise Methyl, Ethyl, Isopropyl, Propyl, Butyl, sek.-Butyl oder tert.-Butyl, ferner auch Pentyl, 1-, 2- oder 3-Methylbutyl, 1,1-, 1,2- oder 2,2-Dimethylpropyl, 1-Ethylpropyl, Hexyl, Heptyl, Octyl, Nonyl, Decyl, Undecyl oder Dodecyl.

Ein geradkettiges oder verzweigtes Alkenyl mit 2 bis 20 C-Atomen, wobei auch mehrere Doppelbindungen vorhanden sein können, ist beispielsweise Allyl, 2- oder 3-Butenyl, Isobutenyl, sek.-Butenyl, ferner 4-Pentenyl, iso-Pentenyl, Hexenyl, Heptenyl, Octenyl, $-C_9H_{17}$, $-C_{10}H_{19}$ bis $-C_{20}H_{39}$; vorzugsweise Allyl, 2- oder 3-Butenyl, Isobutenyl, sek.-Butenyl, ferner bevorzugt ist 4-Pentenyl, iso-Pentenyl oder Hexenyl.

Ein geradkettiges oder verzweigtes Alkinyl mit 2 bis 20 C-Atomen, wobei auch mehrere Dreifachbindungen vorhanden sein können, ist beispielsweise Ethinyl, 1- oder 2-Propinyl, 2- oder 3-Butinyl, ferner 4-Pentinyl, 3-Pentinyl, Hexinyl, Heptinyl, Octinyl, $-C_9H_{15}$, $-C_{10}H_{17}$ bis $-C_{20}H_{37}$, vorzugsweise Ethinyl, 1- oder 2-Propinyl, 2- oder 3-Butinyl, 4-Pentinyl, 3-Pentinyl oder Hexinyl.

Unsubstituierte gesättigte oder teilweise oder vollständig ungesättigte Cycloalkylgruppen mit 5-7 C-Atomen sind daher Cyclopentyl, Cyclohexyl, Cycloheptyl, Cyclopentenyl, Cyclopenta-1,3-dienyl, Cyclohexenyl, Cyclohexa-1,3-dienyl, Cyclohexa-1,4-dienyl, Phenyl, Cycloheptenyl, Cyclohepta-1,3-dienyl, Cyclohepta-1,4-dienyl oder Cyclohepta-1,5-dienyl, welche mit $C_1$- bis $C_6$-Alkylgruppen substituiert sein können.

$HetN^+$ ist bevorzugt

Imidazolium
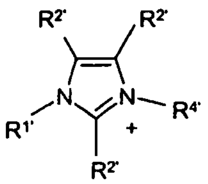
,
Pyrazolium
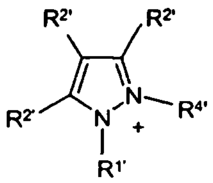
,
Pyrrolidinium
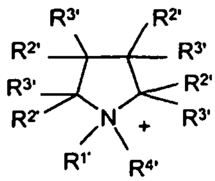
,
Pyridinium
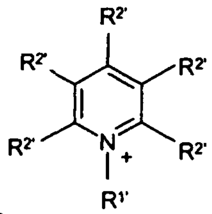
,
Pyrimidinium
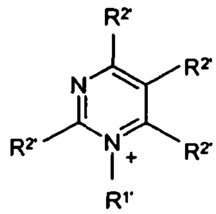
,

Piperidinium
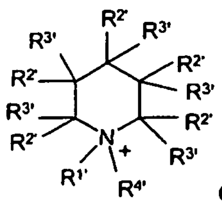
oder
Indolinium
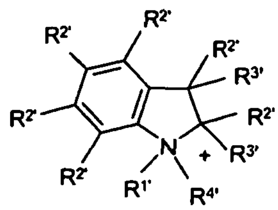
,

wobei die Substituenten $R^{1'}$ bis $R^{4'}$ jeweils unabhängig voneinander eine zuvor beschriebene Bedeutung haben.

Ganz besonders bevorzugt ist $HetN^+$

Imidazolium
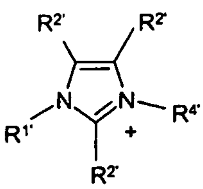
,
Pyrrolidinium
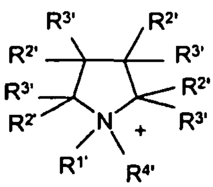
,
Pyridinium
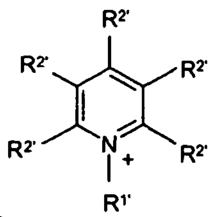
,
Piperidinium
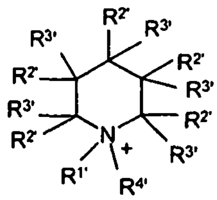

oder,

Morpholinium
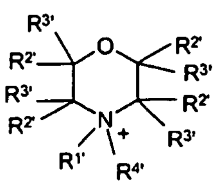
,

wobei die Substituenten $R^{1'}$ bis $R^{4'}$ jeweils unabhängig voneinander eine zuvor beschriebene Bedeutung haben.

$HetN^{z+}$ ist ganz besonders bevorzugt Imidazolium, wobei die Substituenten $R^{1'}$ bis $R^{4'}$ jeweils unabhängig voneinander eine zuvor beschriebene Bedeutung haben.

Nach Anspruch 1 wird die mindestens eine Ionische Flüssigkeit enthaltend Tetracyanoboratanionen ausgewählt aus der Gruppe der Verbindungen der Formeln (1), (2) oder (7), mit Substituenten, wie zuvor definiert. Ganz besonders bevorzugt werden Verbindungen der Formel (7) und deren bevorzugte Verbindungen eingesetzt, wie zuvor beschrieben.

Besonders bevorzugt werden die Ionischen Flüssigkeiten enthaltend Tetracyanoboratanionen für den Einsatz im erfindungsgemäßen Verfahren ausgewählt aus der Gruppe

1-Octyl-3-methylimidazolium Tetracyanoborat,
1-Decyl-3-methylimidazolium Tetracyanoborat,
1-Dodecyl-3-methylimidazolium Tetracyanoborat,
Trihexyl-tetradecyl-ammonium Tetracyanoborat,

Trihexyl-tetradecyl-phosphonium Tetracyanoborat,
N-Octylpyridinium Tetracyanoborat,
1-Octyl-1-methyl-pyrrolidinium Tetracyanoborat,
N-Octyl-N-methyl-morpholinium Tetracyanoborat,
1-Octyl-1-methyl-piperidinium Tetracyanoborat.

Ganz besonders bevorzugt wird 1-Octyl-3-methylimidazolium Tetracyanoborat verwendet. Ganz besonders bevorzugt wird 1-Dodecyl-3-methylimidazolium Tetracyanoborat verwendet.

Der Vorteil der ionischen Flüssigkeit (IL) enthaltend ein Tetracyanoboratanion, beispielsweise von DMIM TCB, gegenüber Oleylakohol (OA) liegt darin, dass die ionische Flüssigkeit Butanol und Aceton parallel extrahieren kann, ohne dass der Verteilungskoeffizient für Butanol eingeschränkt wird. Diese Aussage wird in den Beispielen 2 und 3 belegt. Oleylalkohol ist nicht nennenswert in der Lage Aceton zu extrahieren. Wenn mit OA extrahiert wird und gleichzeitig Aceton in der wässrigen Phase vorliegt, verringert sich der Anteil des extrahierten Butanols, d.h. der Verteilungskoeffizient für Butanol verringert sich durch die Anwesenheit des Acetons. Aus der entstehenden IL-Phase enthaltend Aceton und Butanol können beide Komponenten destillativ voneinander getrennt werden und die beiden Wertstoffe Aceton und Butanol können verwertet werden. Bei Verwendung von Oleylalkohol kann Aceton nicht verwertet werden. Figur 7 zeigt einen Vergleich der Verteilungskoeffizienten für Butanol von DMIM TCB gegenüber Oleylalkohol für Butanol-Wasser (eins), Aceton-Butanol-Wasser (zwei) und Aceton-Butanol-Ethanol (1 Gew.-%, 2 Gew.-%, 0.3 Gew.-%, drei) bei 25°C (siehe Beispiel 6). Der linke Balken entspricht jeweils DMIM TCB, der rechte Balken Oleylalkohol. Die Zahlen eins, zwei, drei geben die Anzahl der Komponenten an.

Es versteht sich für den Fachmann von selbst, dass in den erfindungsgemäßen Verbindungen Substituenten wie beispielsweise C, H, N, O, Cl, F durch die entsprechenden Isotope ersetzt sein können.

Die Bereitstellung der wässrigen Lösung enthaltend mindestens einen Alkohol des erfindungsgemäßen Verfahrens gehört zum allgemeinen Fachwissen. Es kann die wässrige Lösung sowohl gezielt hergestellt werden, als auch eine wässrige Lösung aus einem Produktionsprozess eingesetzt werden. Im besonderen Falle der Fermentationsbrühe ist der Produktionsprozess eine Fermentation.

Eine intensive Mischung kann vorzugsweise unter Rühren stattfinden. Es sind jedoch auch alle anderen Arten von Vermischungen möglich, beispielsweise physikalische Vorgänge wie Schütteln oder Ultraschall.

Das Abtrennen der mindestens einphasigen Mischung aus Schritt b) des erfindungsgemäßen Verfahrens von der wässrigen Lösung erfolgt nach Methoden, die dem Fachmann bekannt sind.
Wird das erfindungsgemäße Verfahren im Batch-Betrieb genutzt, so entsteht nach Beendigung des Rührens eine Entmischung der wässrigen Lösung von der mindestens einphasigen Mischung enthaltend mindestens einen Teil des zu extrahierenden Alkohols, wie zuvor definiert und die mindestens eine Ionische Flüssigkeit und die untere Phase kann beispielsweise durch Entnahme am Boden des Reaktionsgefäßes abgetrennt werden.
Bei kontinuierlicher Nutzung des Verfahrens wird ebenfalls am Boden des Reaktionsgefäßes kontinuierlich ein Teil der unteren Phase abgezogen. Verwiesen wird insbesondere auf das Fachwissen, dokumentiert durch beispielsweise die Fachliteratur von J. Rydberg, M. Cox, C. Muskas, G.R. Chopppin, 2004, Solvent Extraction Principles and Practice oder R. H. Perry, 1984, Perry's chemical engineer's handbook.

Die untere Phase in diesem Abtrennprozess ist in der Regel die mindestens mehrphasige Mischung der mindestens einen Ionischen Flüssigkeit mit dem extrahierten Teil des Alkohols, wie zuvor beschrieben.

Die Abtrennung der Komponente Alkohol von der Ionischen Flüssigkeit erfolgt nach Methoden, die dem Fachmann bekannt sind, beispielweise durch Destillation des Alkohols, Strippen, Flash, Verdampfung, Adsorption oder chromatographischen Methoden.

Die aufbereitete Ionische Flüssigkeit, wie zuvor beschrieben, kann gegebenenfalls in das erfindungsgemäße Verfahren zurückgeführt werden und steht wieder als Lösungsmittel zur Verfügung.

Das erfindungsgemäße Verfahren zur Flüssig-Flüssig-Extraktion kann im kontinuierlichen aber auch im halbkontinuierlichen Betrieb durchgeführt werden, wie zuvor beschrieben. Die Aufreinigung der Flüssig-Flüssig-Extraktion kann dabei sowohl in-situ als auch entkoppelt stattfinden. Bei der in-situ-Extraktion wird gleichzeitig fermentiert und der Wertstoff, hier erfindungsgemäß mindestens ein Alkohol, wie zuvor beschrieben, abgetrennt, in dem die Fermentationsbrühe direkt mit der Ionischen Flüssigkeit in Kontakt gebracht wird. Somit wird der Wertstoff aus der wässrigen Phase entfernt und die Konzentration des Wertstoffes niedrig gehalten, so dass dieser die Mikroorganismen nicht inhibiert. Inhibierung bedeutet, dass der Wachstum der Mikroorganismen verlangsamt oder gar gestoppt wird, wodurch auch die Produktion des Wertstoffes verlangsamt oder gar gestoppt wird.

Das erfindungsgemäße Verfahren, wie zuvor beschrieben, kann in jeder geeigneten Apparatur durchgeführt werden, wie Sie dem Fachmann bekannt sind.

Bevorzugte Merkmalskombinationen der Erfindung sind in den Ansprüchen offenbart.

Auch ohne weitere Ausführungen wird davon ausgegangen, dass ein Fachmann die obige Beschreibung im weitesten Umfang nutzen kann. Die bevorzugten Ausführungsformen und Beispiele sind deswegen lediglich als beschreibende, keineswegs als in irgendeiner Weise limitierende Offenbarung aufzufassen.

**Beispiele:**

Die Synthese der Ionischen Flüssigkeiten enthaltend

Tetracyanoboratanionen kann beispielsweise nach der Offenbarung der WO 2004/072089 ("Salze mit Cyanoborat-Anionen, Merck Patent GmbH) erfolgen oder sind kommerziell erhältlich.

Beispiele zur Synthese ausgewählter Verbindungen sind:

Beispiel A: Synthese von Trihexyl-tetradecyl-ammonium Tetracyanoborat

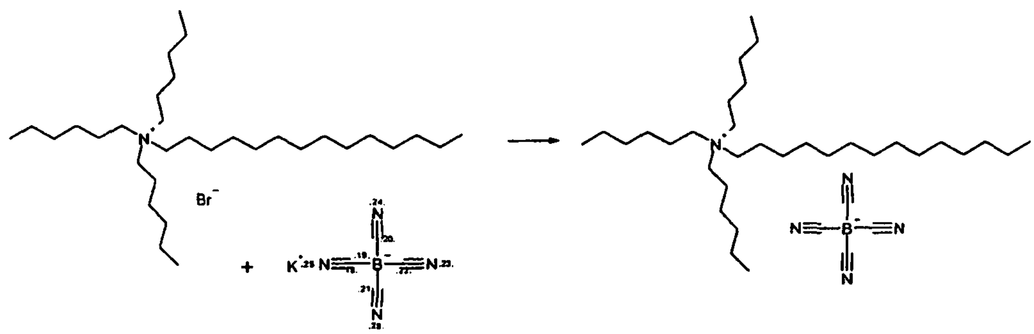

Es werden 100 g Trihexyl-tetradecyl-ammonium bromid in 400 L VE-Wasser gelöst und anschließend langsam mit 31 g Kaliumtetracyanoborat versetzt. Es bilden sich nach und nach 2 Phasen. Dieses zwei-PhasenGemisch wird noch für weitere 2 Stunden (h) bei Raumtemperatur nachgerührt und über Nacht stehen gelassen.

Die Aufarbeitung erfolgt durch Extraktion mit Dichlormethan. Die organische Phase wird dann mit VE-Wasser Bromid-frei gewaschen. Die organische Lösung wird mit 8 g ALOX und 5 g Aktivkohle versetzt, filtriert und anschließend am Rotationsverdampfer bei einem Wasserbad von ca. 80 °C eingeengt. Man erhält eine klare, gelbliche, viskose Flüssigkeit.

Beispiel B: Synthese von Trihexyl-tetradecyl-phosphonium Tetracyanoborat

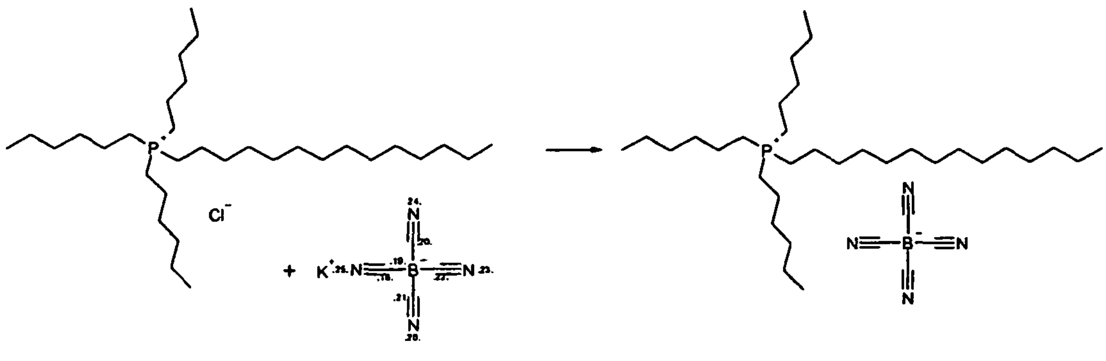

Analog zu Beispiel A werden 100 g Trihexyl-tetradecyl-phosphonium chlorid mit 33g Kaliumtetracyanoborat versetzt und entsprechend aufgearbeitet. Man erhält eine klare, gelbliche, viskose Flüssigkeit.

Beispiel C: Synthese von 1-Methyl-1-octylpyrrolidinium Tetracyanoborat

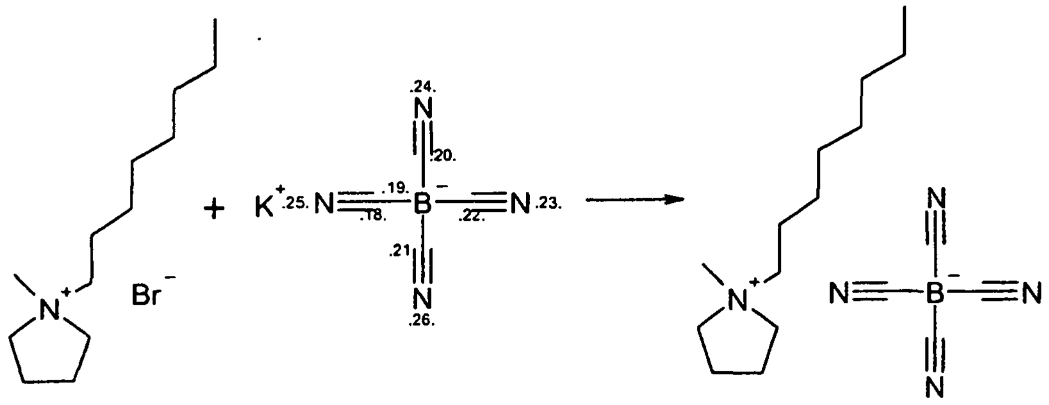

Analog zu Beispiel A werden 100 g 1-Methyl-1-octyl-pyrrolidinium bromid mit 61 g Kaliumtetracyanoborat versetzt und entsprechend aufgearbeitet. Man erhält eine klare, gelbliche, viskose Flüssigkeit.

1 H NMR (d6-DMSO): δ = 3.42 (m, 4H), 3.27 (m, 4H), 2.97 (s, 3H), 2.50 (m, 2H) 1.69 (m, 2H), 1.23 (m, 10H), 0.87 (t, *J*(H,H) = 7.1 Hz, 3H).

Beispiel D: Synthese von N-Methyl-N-octylmorpholinium Tetracyanoborat

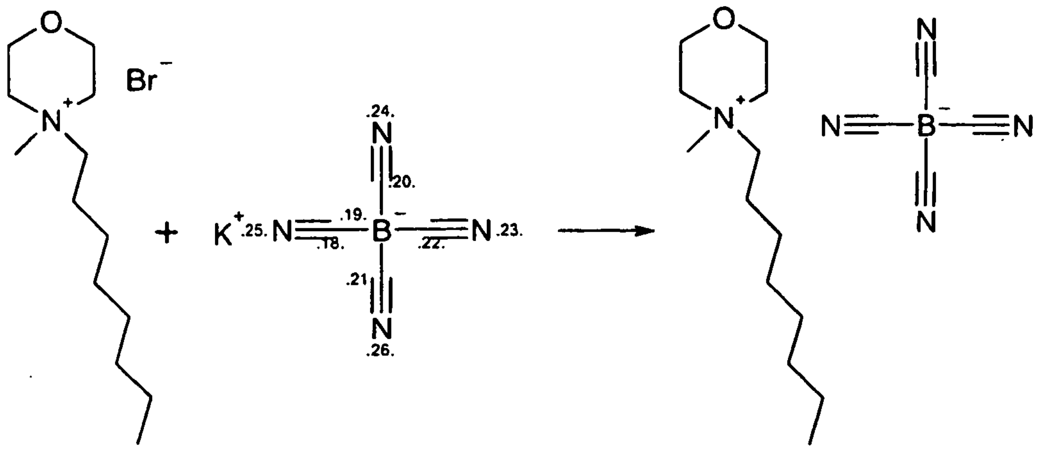

Analog zu Beispiel A werden 100 g 4-Methyl-4-octyl-morpholinium bromid mit 58g Kaliumtetracyanoborat versetzt und entsprechend aufgearbeitet. Man erhält eine klare, gelbliche, viskose Flüssigkeit.

1 H NMR (d6-DMSO): δ = 3.90 (m, 4H), 3.39 (m, 6H), 3.10 (s, 3H), 1.67 (m, 2H), 1.29 (m, 10H), 0.87 (t, *J*(H, H) = 7.2 Hz, 3H).

Beispiel E: Synthese von 1-Methyl-1-octylpiperidinium Tetracyanoborat

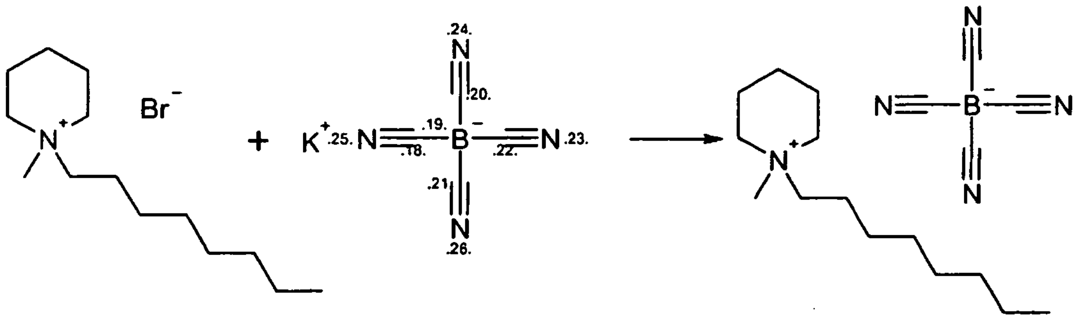

Analog zu Beispiel A werden 100 g 1-Methyl-1-octyl-piperidinium bromid mit 58g Kaliumtetracyanoborat versetzt und entsprechend aufgearbeitet. Man erhält eine klare, gelbliche, viskose Flüssigkeit.

1 H NMR (d6-DMSO): δ = 3.35 (m, 6H), 3.29 (m, 6H), 2.98 (s, 3H), 1.78 (m, 2H), 1.30 (m, 10H), 0.89 (t, *J*(H, H) = 7.2 Hz, 3H).

Beispiel F: Synthese von 1-Octyl-3-methyl-imidazolium Tetracyanoborat

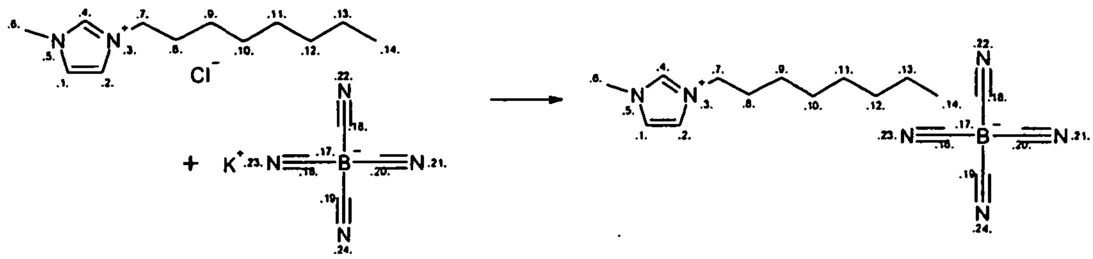

Analog zu Beispiel A werden 250 g 3-Methyl-1-octyl-imidazolium chlorid mit 183g Kaliumtetracyanoborat versetzt und entsprechend aufgearbeitet. Man erhält eine klare, leicht gelbliche Flüssigkeit.

1 H NMR (d6-DMSO): δ = 9.10 (s, 1 H), 7.69 (t, *J*(H,H) = 1.8 Hz, 1 H), 7.63 (t, *J*(H,H) = 1.8 Hz, 1H), 4.16 (t, *J* (H,H) = 7.4 Hz, 2H), 3.86 (s, 3H), 1.81 (m, 2H), 1.29 (m, 10H), 0.87 (t, *J*(H,H) = 6.9 Hz, 3H).

Beispiel G: Synthese von N-Octylpyridinium Tetracyanoborat

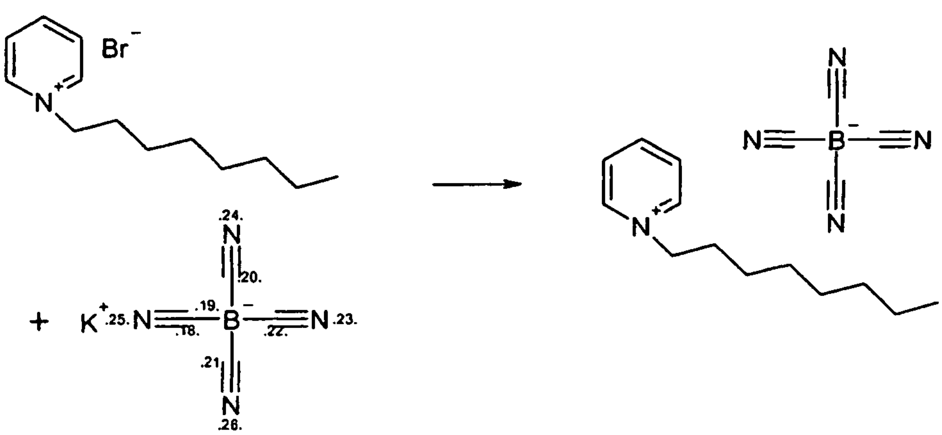

Analog zu Beispiel A werden 134g 1-Octylpyridinium bromid mit 83g Kaliumtetracyanoborat versetzt und entsprechend aufgearbeitet. Man erhält eine klare, gelbliche, viskose Flüssigkeit.

1 H NMR (d6-DMSO): δ = 9.09 (m, 2H), 8.61 (m, 1 H), 8.16 (m, 2H), 4.60 (t, *J*(H,H) = 7.5 Hz, 2H), 3.31 (m,

2H), 2.50 (m, 2H), 1.93 (m, 2H), 1.23 (m, 6H), 1.23 (t, *J*(H,H) = 7.2 Hz, 3H).

**Beispiel 1:**

**Durchführung:**

Die Messung des Verteilungskoeffizienten erfolgt in doppelwandigen Glassgefäßen mit einem maximalen Volumen von 10 ml. Die Anfangskonzentration von Butanol in der wässrige Phase ist 1 Gew.%. Gleiche Massen (3 g) von jeder Phase werden in Kontakt gebracht und mittels eines Magnetrührers (Variomag telesystem 06.07) bei konstanter Temperatur (25°C) 24 h intensiv vermischt. Durch die lange Versuchsdauer wird das Erreichen des Gleichgewichts gewährleistet. Die Temperierung erfolgt mittels eines Kryostaten (Julabo F25 ME). Nach 10 Minuten Phasentrennung werden Proben von jeder Phase entnommen und analysiert.
Die Ergebnisse für die Ionischen Flüssigkeiten [1] bis [7], wie später gelistet, sind in Figur 3 graphisch dargestellt.

Die Selektivität S von n-Butanol gegenüber Wasser ist gegen den Verteilungskoeffizienten D von n-Butanol aufgetragen. Alle untersuchten Ionischen Flüssigkeiten haben einen Verteilungskoeffizienten größer bzw. gleich 2 und die Selektivitäten sind alle größer 73. Die Ergebnisse belegen daher die besondere Eignung als Lösungsmittel für n-Butanol aus wässrigen Lösungen.
Die ionischen Flüssigkeiten [1] und [2] zeigen aufgrund der längeren Alkylketten hydrophoberes Verhalten und haben somit viel höhrere Selektivitäten verglichen mit den anderen untersuchten ionischen Flüssigkeiten. Die ionische Flüssigkeiten [3] und [7] zeigen fast gleiche Verteilungskoeffizienten von 3 und geringfügige Unterschiede in der Selektivität. Den höchsten uns somit besten Verteilungskoeffizient von 3,7 hat die ionische Flüssigkeit [6] mit einer Selektivität von 97.

Die zur Zeit am meisten untersuchte Substanz Oleylalkohol erreicht in dieser Untersuchung einen Verteilungskoeffizienten von 3,4 und eine Selektivität von 208.

[1] Trihexyl-tetradecyl-ammonium Tetracyanoborat
[2] Trihexyl-tetradecyl-phosphonium Tetracyanoborat
[3] 1-Methyl-1-octyl-pyrrolidinium Tetracyanoborat
[4] N-Octyl-N-methyl-morpholinium Tetracyanoborat
[5] 1-Methyl-1octyl-piperidinium Tetracyanoborat
[6] 1-Octyl-3-methyl-imidazolium Tetracyanoborat
[7] 1-Octyl-pyridinium Tetracyanoborat.

Beispiel 2:

Die Messung der Verteilungskoeffizienten erfolgt in doppelwandigen Glassgefäßen mit einem maximalen Volumen von 10 ml. Es werden zwei verschiedene wässrige Phasen eingesetzt. Die wässrige Phase A beinhaltet neben Wasser nur die Komponenten Butanol in einer Konzentration von 2 Gew.%. Die wässrige Phase B enthält zusätzlich noch die Komponente Aceton. Die Anfangskonzentrationen dieser Phase B sind 2 Gew.% von Butanol und 1 Gew.% von Aceton. Untersucht wird die IL 1-Decyl-3-methyl-imidazolium Tetracyanoborat. Gleiche Massen (3g) von jeder Phase werden in Kontakt gebracht und mittels eines Magnetrührers (Variomag telesystem 06.07) bei konstanter Temperatur (25° C) 24 h intensiv vermischt. Die Temperierung erfolgt mittels eines Kryostaten (Julabo F25 ME). Nach 10 Minuten Phasentrennung werden Proben von jeder Phase entnommen und analysiert.
Die Selektivität S gegenüber Wasser wird nicht durch die Anwesenheit der weiteren Komponente Aceton beeinflusst. Die Selektivität S von n-Butanol gegenüber Wasser beträgt für die wässrige Phase A 104 und für die wässrige Phase B 100.
Der Verteilungskoeffizient D von n-Butanol bei der Extraktion aus der wässrigen Phase A beträgt 3,27. Der Verteilungskoeffizient D von n-Butanol bei der Extraktion aus der wässrigen Phase B beträgt 3,23 und der von Aceton 2,36.

Diese Ergebnisse zeigen, dass der Verteilungskoeffizient D von n-Butanol von der Anwesenheit einer weiteren Komponente wie Aceton nicht beeinflusst wird. Der Verteilungskoeffizient D von Aceton in Anwesenheit von Butanol zeigt, dass auch Aceton von der ionischen Flüssigkeit 1-Decyl-3-methyl-imidazolium Tetracyanoborat (DMIM TCB) extrahiert wird, jedoch deutlich weniger als Butanol. Die ionischen Flüssigkeit 1-Decyl-3-methyl-imidazolium Tetracyanoborat kann demzufolge n-Butanol selektiver abtrennen.

Beispiel 3:

Analog zu Beispiel 2 wird bei einer Temperatur von 20°C eine wässrige Phase enthaltend 2 Gew.% n-Butanol, 1 Gew.% Aceton und 0.3 Gew.% Ethanol untersucht. Der Verteilungskoeffizient D von n-Butanol bei 20°C aus der wässrigen Phase für die ionische Flüssigkeit 1-Decyl-3-methyl-imidazolium Tetracyanoborat beträgt 3,04. Die Extraktion

von n-Butanol in Gegenwart von Aceton und Ethanol wird nicht beeinträchtigt und ist somit selektiv möglich.
Beispiele 2 und 3 stützen das erfindungsgemäße Verfahren für ein Zweikomponenten- bzw. Dreikomponentensystem, d.h. einfache Systeme einer synthetischen Fermentationsbrühe einer ABE-Fermentation.

Beispiel 4: Untersuchungen zur Initialkonzentration von Butanol und der Temperatur

Der Einfluss der Temperatur und der Anfangskonzentration von Butanol wird mit der ionischen Flüssigkeit 1-Decyl-3-methyl-imidazolium Tetracyanoborat untersucht. Die Messung des Verteilungskoeffizienten erfolgt in doppelwandigen Glassgefäßen mit einem maximalen Volumen von 10 ml und gleiche Massen (3 g) jeder Phase werden untersucht.. Die Anfangskonzentration von Butanol in der wässrige Phase variiert zwischen 0,2 und 2 Gew.%. Die Temperaturen werden zwischen 20°C und 40°C variiert in Schritten von 5°C. Dies sind gängige Bedingungen einer Fermentation. Die gleichen Massen (3 g) von jeder Phase werden in Kontakt gebracht und mittels eines Magnetrührers (Variomag telesystem 06.07) intensiv vermischt. Die jeweilig untersuchte Temperatur wird durch ein thermostatisches zirkulierendes Wasserbad konstant gehalten (Julabo F25 ME). Nach 10 Minuten Phasentrennung werden Proben von jeder Phase entnommen und analysiert.

Auswertung:

Mit steigender Butanolanfangskonzentration in der wässrigen Phase bei konstanter Temperatur von 25°C erhöht sich der Verteilungskoeffizient von 2.6 auf 3.3. Bei den gemessenen Temperaturen (20°C, 25°, 30°C, 35°C, 40°C) erhöht sich der Verteilungskoeffizient mit ansteigender Anfangskonzentration von Butanol um 0,6 bis 1. Je höher die Temperatur der Extraktion, desto niedriger ist die Erhöhung des Verteilungskoeffizienten.
Mit steigender Temperatur und konstanter Anfangskonzentration von Butanol in der wässrigen Phase von 1 Gew.-%, erhöht sich der Verteilungskoeffizient von 2.7 auf 4.3. Bei jeder gemessenen Anfangskonzentration von Butanol erhält man eine Erhöhung zwischen 1.4 und 3.0 des Verteilungskoeffizienten.
Figur 4 fasst dieses Ergebnis zusammen und beschreibt den Einfluß von Temperatur und Anfangskonzentration auf den Verteilulngskoeffizienten von Butanol im System DMIM TCB/Wasser.
Beobachtet wird ebenfalls eine Erhöhung der Selektivität von Butanol gegen Wasser bei einer konstanten Temperatur von 25°C mit steigender Anfrangskonzentration von 87 auf 107. Mit Erhöhung der Temperatur und konstanter Anfangskonzentration von Butanol in der wässrigen Phase von 1 Gew.-% erhöht sich die Selektivität von 100 auf 133. Bei jeder gemessenen Anfarigskonzentration von Butanol erhält man eine Erhöhung zwischen 28 und 50 der Selektivität.
Figur 5 fasst dieses Ergebnis zusammen und beschreibt den Einfluß von Temperatur und Anfangskonzentration auf die Selektivität von Butanol gegenüber Wasser im System DMIM TCB/Wasser.
Figur 6 zeigt den Einfluß der Temperatur auf die Selektivität von Butanol gegenüber Wasser, aufgezeigt gegen den Verteilungskoeffizienten von Butanol im System DMIM TCB/Wasser für 1 Gew.-% Butanol in der wässrigen Lösung im Vergleich mit Oleylalkohol bei 25°C.

Beispiel 5:

Absetzzeit und Phasentrennung

Gleiche Volumina (3 ml) von Wasser und der ionischen Flüssigkeit 1-Decyl-3-methyl-imidazolium Tetracyanoborat oder von Wasser und Oleylalkohol werden in Kontakt gebracht und intensiv vermischt. Nach Beenden der Durchmischung wird die Zeit zur Phasentrennung gemessen. Nach 15 Minuten erhält man eine klare Phasentrennung im System ionische Flüssigkeit (IL)/Wasser und die IL-Phase hat sich zu 90% ausgebildet.
Nach 15 Minuten hat sich die Oleylalkohol-Phase lediglich zu 35% gebildet, nach 20 Minuten zu 45% und nach 25 Minuten zu 90%.
Die Absetzzeit des IL/Wasser-Systems benötigt demzufolge nur die Hälfte der Zeit im Vergleich zum Oleylalkohol/Wasser-System.

Beispiel 6: Vergleich mit Oleylalkohol

Wie zuvor beschrieben finden sich in der Fermentationsbrühe kleine Mengen an polaren Nebenprodukten wie Ethano oder Aceton. Deshalb ist es relevant, die Leistungsfähigkeit des Extraktionsmittels auch in Gegenwart dieser Nebenprodukte zu überprüfen (siehe auch Beispiele 2 und 3).

Vergleich DMIM TCB mit Oleylalkohol:

Die Messung des Verteilungskoeffizienten erfolgt in doppelwandigen Glassgefäßen mit einem maximalen Volumen von 10 ml und gleiche Massen (3 g) von DMIM TCB oder Oleylalkohol werden untersucht.

Drei Lösungen werden untersucht:

Lösung A enthaltend 2 Gewichtsprozent Butanol (eine Komponente);
Lösung B enthaltend 2 Gewichtsprozent Butanol und 1 Gewichtsprozent Aceton (zwei Komponenten);
Lösung C enthaltend 2 Gewichtsprozent Butanol, 1 Gewichtsprozent Aceton und 0,3 Gewichtsprozent Ethanol.

Die gleichen Massen (3 g) werden mit der jeweiligen Lösung in Kontakt gebracht und mittels eines Magnetrührers (Variomag telesystem 06.07) intensiv vermischt. Die Temperatur wird durch ein thermostatisches zirkulierendes Wasserbad 24 h bei 25°C konstant gehalten (Julabo F25 ME). Nach 10 Minuten Phasentrennung werden Proben von jeder Phase entnommen und analysiert.

Für die Lösung A ist der Verteilungskoeffizient von Butanol für DMIM TCB 3.2, für Oleylalkohol (OA) 4.0.

Für die Lösung B ist der Verteilungskoeffizient von Butanol für DMIM TCB auf 3.5 angestiegen, währen der Verteilungskoeffizient von Butanol für OA auf 4.0 sinkt.

Für die Lösung C ist der Verteilungskoeffizient von Butanol für DMIM TCB statistisch gesehen vergleichbar zu dem Wert für OA. Figur 7 veranschlaulicht dieses Ergebnis.

Die Anwesenheit von polaren Komponenten wie beispielsweise Aceton oder Ethanol verbessert die Leistungsfähigkeit von DMIM TCB. Die gleichen polaren Komponenten bewirken einen Abfall der Leistungsfähigkeit von Oleylalkohol.

**Verzeichnis der Figuren:**

Figur 1: Variante einer Flüssig-Flüssig-Extraktion
Figur 2: Fermentation mit Hilfe einer Extraktionskolonne
Figur 3: Selektivität S von n-Butanol gegenüber Wasser ist gegen den Verteilungskoeffizienten D von n-Butanol aufgetragen
Figur 4: Einfluß der Temperatur und Anfangskonzentration auf den Verteilungskoeffizienten D von Butanol im System DMIM TCB/Wasser
Figur 5: Einfluß der Temperatur und Anfangskonzentration auf die Selektivität von Butanol gegenüber Wasser im System DMIM TCB/Wasser
Figur 6: Einfluß der Temperatur auf die Selektivität von Butanol gegenüber Wasser aufgetragen gegen den Verteilungskoeffizienten D von Butanol im System DMIM TCB/Wasser mit 1 Gew.-% Anfangskonzentration von Butanol.
Figur 7: Vergleich der Verteilungskoeffizienten für Butanol von DMIM TCB gegenüber Oleylalkohol für Butanol-Wasser (eins), Aceton-Butanol-Wasser (zwei) und Aceton-Butanol-Ethanol (1 Gew.-%, 2 Gew.-%, 0.3 Gew.-%; drei) bei 25°C.

**Patentansprüche**

**1.** Verfahren zur Flüssig-Flüssig-Extraktion von Alkoholen aus wässrigen Lösungen, wobei

a) die wässrige Lösung enthaltend mindestens einen Alkohol bereitgestellt wird und die wässrige Löung den Alkohol in einer Konzentrationvon 0.5 bis 10 Gewichtsprozent bezogen auf die wässrige Lösung enthält,
b) die wässrige Lösung aus a) mit einer mindestens einen Ionischen Flüssigkeit enthaltend Tetracyanoboratanionen intensiv gemischt wird, so dass die Ionische Flüssigkeit in der Lage ist, mindestens einen Teil des Alkohols aus der wässrigen Lösung zu extrahieren und mit diesem Alkohol eine mindestens einphasige Mischung herzustellen,
c) abtrennen der mindestens einphasigen Mischung aus b) von der wässrigen Lösung,
d) trennen der einphasigen Mischung aus b) in die Komponenten Alkohol und Ionische Flüssigkeit, und gegebenenfalls
e) zurückführen der Ionischen Flüssigkeit aus d) in Schritt b), wobei die mindestens eine Ionische Flüssigkeit enthaltend Tetracyanoboratanionen ausgewählt wird aus der Gruppe der Verbindungen der Formeln (1) oder (2),

$[NR_4]^+ [B(CN)_4]^-$ (1),

$[PR_4]^+ [B(CN)_4]^-$ (2),

wobei
R jeweils unabhängig voneinander

- ein geradkettiges oder verzweigtes Alkyl mit 1-20 C-Atomen,
- ein geradkettiges oder verzweigtes Alkenyl mit 2-20 C-Atomen und einer oder mehreren Doppelbindungen,
- ein geradkettiges oder verzweigtes Alkinyl mit 2-20 C-Atomen und einer oder mehreren Dreifachbindungen,
- gesättigtes, teilweise oder vollständig ungesättigtes Cycloalkyl mit 5-7 C-Atomen, das mit Alkylgruppen mit 1-6 C-Atomen substituiert sein kann, bedeutet, mit der Bedingung, dass mindestens zwei Substituenten R mindestens 5 C-Atome haben

oder
ausgewählt wird aus der Gruppe der Verbindungen der Formel (7),

$[HetN]^+ [B(CN)_4]^-$ (7),

wobei
- $HetN^+$ ein heterocyclisches Kation, ausgewählt aus der Gruppe

Imidazolium

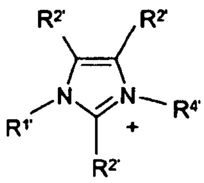

,

1H-Pyrazolium

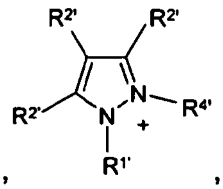

,

3H-Pyrazolium

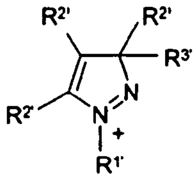

,

4H-Pyrazolium

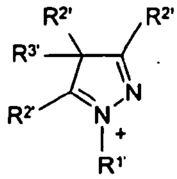

,

1-Pyrazolinium

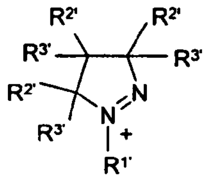

,

2-Pyrazolinium

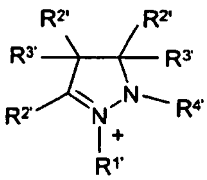

,

3-Pyrazolinium

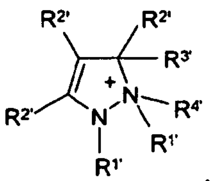

,

2,3-Dihydro-Imidazolinium

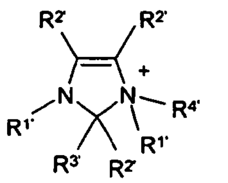

,

4,5-Dihydro-Imidazolinium

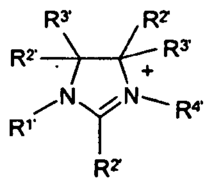

,

2,5-Dihydro-Imidazolinium

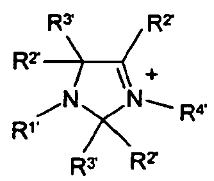

,

Pyrrolidinium

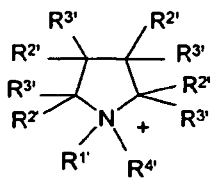

,

1,2,4-Triazolium

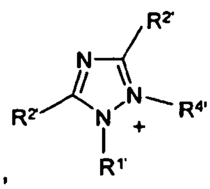

,

1,2,4-Triazolium

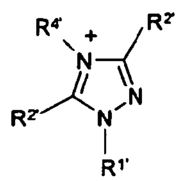

,

1,2,3-Triazolium
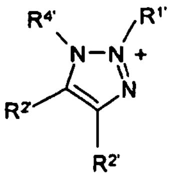
,
1,2,3-Triazolium
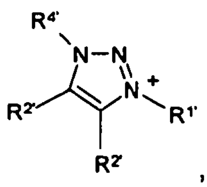
,
Pyridinium
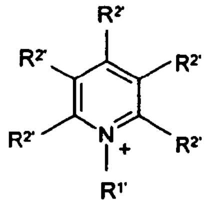
,
Pyridazinium
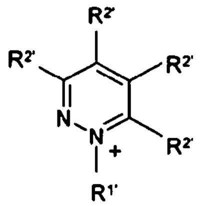
,
Pyrimidinium
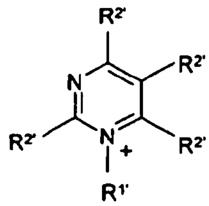
,

Piperidinium
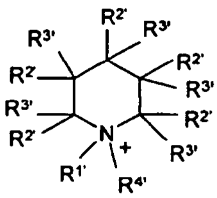
,
Morpholinium
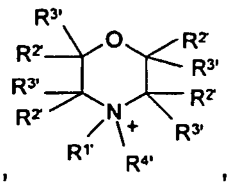
,
Piperazinium
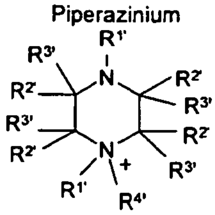
,
Piperazinium
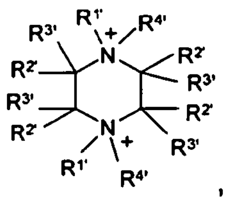
,

Pyrazinium
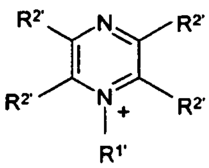
,
Thiazolium
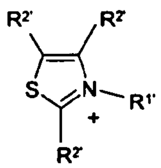
,
Oxazolium
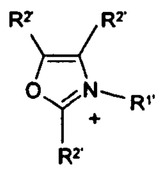
,
Indolium
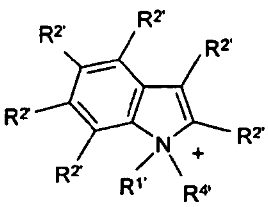
,,

Chinolinium
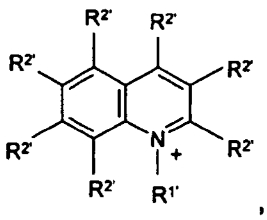
,
Isochinolinium
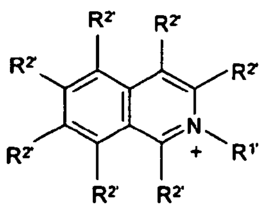
,
Chinoxalinium
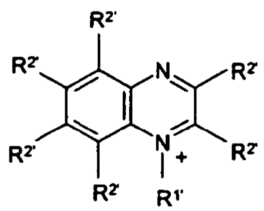

oder

Indolinium
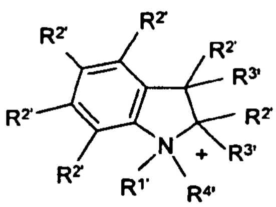

bedeutet, wobei die Substituenten
$R^{1'}$ bis $R^{4'}$ jeweils unabhängig voneinander

- ein geradkettiges oder verzweigtes Alkyl mit 1-20 C-Atomen,
- ein geradkettiges oder verzweigtes Alkenyl mit 2-20 C-Atomen und einer oder mehreren Doppelbindungen,
- ein geradkettiges oder verzweigtes Alkinyl mit 2-20 C-Atomen und einer oder mehreren Dreifachbindungen,
- gesättigtes, teilweise oder vollständig ungesättigtes Cycloalkyl mit 5-7 C-Atomen, das mit Alkylgruppen mit 1-6 C-Atomen substituiert sein kann, bedeutet,

wobei die Substituenten $R^{1'}$, $R^{2'}$, $R^{3'}$ und/oder $R^{4'}$ zusammen auch ein Ringsystem bilden können und wobei der mindestens eine Alkohol aus der Gruppe Ethanol, Isopropanol, Propanol, n-Butanol oder Isomere des n-Butanol oder Mischungen derselben gewählt wird.

**2.** Verfahren nach Anspruch 1, **dadurch gekennzeichnet, dass** das Verfahren kontinuierlich oder halbkontinuierlich durchgeführt wird.

**3.** Verfahren nach Anspruch 1, **dadurch gekennzeichnet, dass** der mindestens eine Alkohol n-Butanol ist.

**4.** Verfahren nach einem oder mehreren der Ansprüche 1 bis 3, **dadurch gekennzeichnet, dass** die wässrige Lösung enthaltend mindestens einen Alkohol eine Fermentationsbrühe ist.

**5.** Verfahren nach einem oder mehreren der Ansprüche 1 bis 4, **dadurch gekennzeichnet, dass** die Fermentationsbrühe aus einer Aceton-Butanol-Ethanol-Fermentation stammt.

**6.** Verfahren nach einem oder mehreren der Ansprüche 1 bis 5, **dadurch gekennzeichnet, dass** die mindestens eine Ionische Flüssigkeit enthaltend Tetracyanoboratanionen ausgewählt wird aus der Gruppe
1-Octyl-3-methyl-imidazolium Tetracyanoborat,
1-Decyl-3-methyl-imidazolium Tetracyanoborat,
1-Dodecyl-3-methyl-imidazolium Tetracyanoborat,
Trihexyl-tetradecyl-ammonium Tetracyanoborat,
Trihexyl-tetradecyl-phosphonium Tetracyanoborat,
N-Octylpyridinium Tetracyanoborat,
1-Octyl-1-methyl-pyrrolidinium Tetracyanoborat,
N-Octyl-N-methyl-morpholinium Tetracyanoborat,
1-Octyl-1-methyl-piperidinium Tetracyanoborat.

## Claims

**1.** Method for the liquid-liquid extraction of alcohols from aqueous solutions, where

a) the aqueous solution comprising at least one alcohol is provided and the aqueous solution comprises the alcohol in a concentration of 0.5 to 10 per cent by weight, based on the aquous solution,
b) the aqueous solution from a) is mixed intensively with at least one ionic liquid containing tetracyanoborate anions, so that the ionic liquid is able to extract at least some of the alcohol from the aqueous solution and form an at least single-phase mixture with this alcohol,
c) the at least single-phase mixture from b) is separated off from the aqueous solution,
d) the single-phase mixture from b) is separated into the components alcohol and ionic liquid, and optionally
e) the ionic liquid from d) is fed back into step b),
where the at least one ionic liquid containing tetracyanoborate anions is selected from the group of the compounds of the formulae (1) and (2),

$$[NR_4]^+ [B(CN)_4]^- \qquad (1),$$

$$[PR_4]^+ [B(CN)_4]^- \qquad (2),$$

where

R in each case, independently of one another, denotes

- a straight-chain or branched alkyl having 1-20 C atoms,
- a straight-chain or branched alkenyl having 2-20 C atoms and one or more double bonds,
- a straight-chain or branched alkynyl having 2-20 C atoms and one or more triple bonds,
- saturated, partially or fully unsaturated cycloalkyl having 5-7 C atoms, which may be substituted by alkyl groups having 1-6 C atoms,

with the proviso that at least two substituents R have at least 5 C atoms,
or
is selected from the group of the compounds of the formula (7),

$[HetN]^+ [B(CN)_4]^-$ (7),

where
$HetN^+$ denotes a heterocyclic cation selected from the group

imidazolium
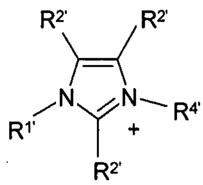
,
1H-pyrazolium
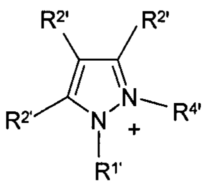
,
3H-pyrazolium
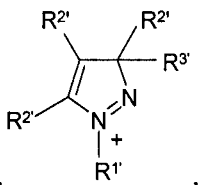
,
4H-pyrazolium
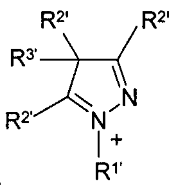
,
1-pyrazolinium
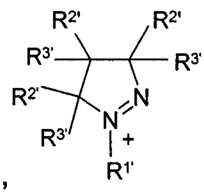
,

2-pyrazolinium
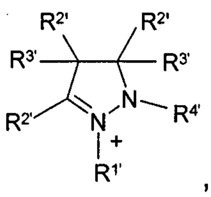
,
3-pyrazolinium
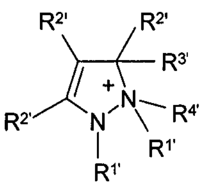
,
2,3-dihydroimidazolinium
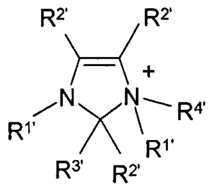
,
4,5-dihydroimidazolinium
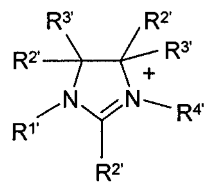
,

2,5-dihydroimidazolinium
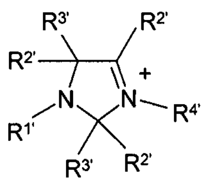
,
pyrrolidinium
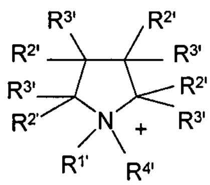
,
1,2,4-triazolium
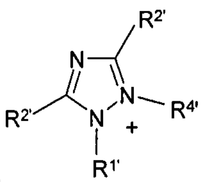
,
1,2,4-triazolium
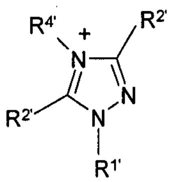
,

1,2,3-triazolium
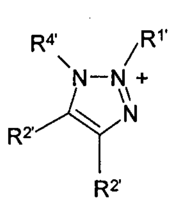
,
1,2,3-triazolium
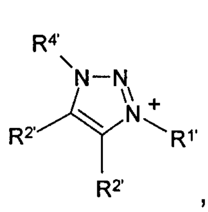
,
pyridinium
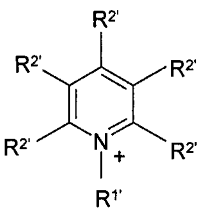
,
pyridazinium
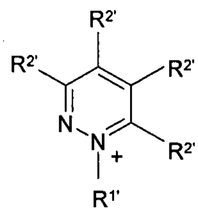
,
pyrimidinium
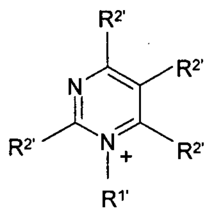
,

piperidinium

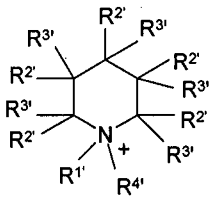

,

morpholinium

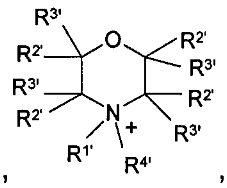

,

piperazinium

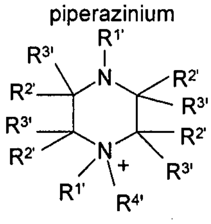

,

piperazinium

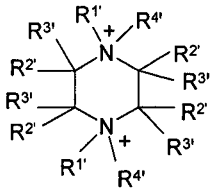

,

pyrazinium

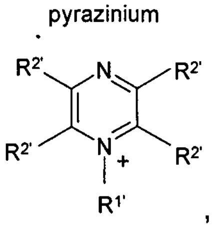

,

thiazolium

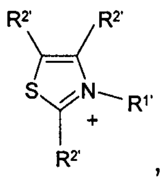

,

oxazolium

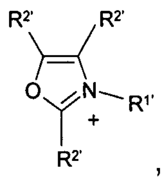

,

indolium

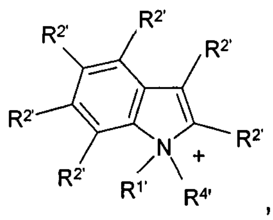

,,

quinolinium

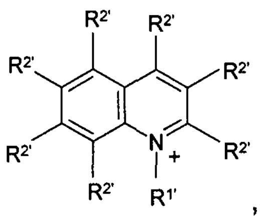

,

isoquinolinium

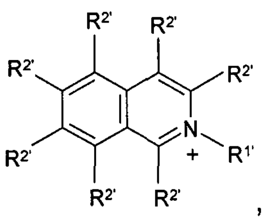

,

quinoxalinium

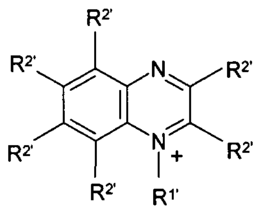

or

indolinium

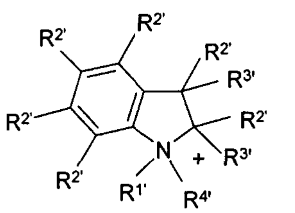

where the substituents
$R^{1,}$ to $R^{4'}$ each, independently of one another, denote

- a straight-chain or branched alkyl having 1-20 C atoms,
- a straight-chain or branched alkenyl having 2-20 C atoms and one or more double bonds,
- a straight-chain or branched alkynyl having 2-20 C atoms and one or more triple bonds,
- saturated, partially or fully unsaturated cycloalkyl having 5-7 C atoms, which may be substituted by alkyl groups having 1-6 C atoms,

where the substituents $R^{1'}$, $R^{2'}$, $R^{3'}$ and/or $R^{4'}$ together may also form a ring system and where the at least one alcohol is selected from the group ethanol, isopropanol, propanol, n-butanol or isomers of n-butanol, or mixtures thereof.

2. Method according to Claim 1, **characterised in that** the method is carried out continuously or semicontinuously.

3. Method according to Claim 1, **characterised in that** the at least one alcohol is n-butanol.

4. Method according to one or more of Claims 1 to 3, **characterised in that** the aqueous solution comprising at least one alcohol is a fermentation broth.

5. Method according to one or more of Claims 1 to 4, **characterised in that** the fermentation broth originates from an acetone-butanol-ethanol fermentation.

6. Method according to one or more of Claims 1 to 5, **characterised in that** the at least one ionic liquid containing tetracyanoborate anions is selected from the group
1-octyl-3-methylimidazolium tetracyanoborate,
1-decyl-3-methylimidazolium tetracyanoborate,
1-dodecyl-3-methylimidazolium tetracyanoborate,
trihexyltetradecylammonium tetracyanoborate,
trihexyltetradecylphosphonium tetracyanoborate,
N-octylpyridinium tetracyanoborate,
1-octyl-1-methylpyrrolidinium tetracyanoborate,
N-octyl-N-methylmorpholinium tetracyanoborate,
1-octyl-1-methylpiperidinium tetracyanoborate.

**Revendications**

1. Méthode pour l'extraction liquide-liquide d'alcools à partir de solutions aqueuses, dans laquelle

a) la solution aqueuse comprenant au moins un alcool est fournie et la solution aqueuse comprend l'alcool selon une concentration allant de 0,5 à 10% en poids, sur la base de la solution aqueuse,
b) la solution aqueuse issue de a) est mélangée vigoureusement avec au moins un liquide ionique contenant des anions tétracyanoborate, de sorte que le liquide ionique soit capable d'extraire au moins une partie de l'alcool à partir de la solution aqueuse et forme un mélange au moins monophasique avec cet alcool,
c) le mélange au moins monophasique issu de b) est séparé de la solution aqueuse,
d) le mélange monophasique issu de b) est séparé selon les composants alcool et liquide ionique, et éventuellement
e) le liquide ionique issu de d) est réalimenté dans l'étape b),

où le au moins un liquide ionique contenant des anions tétracyanoborate est choisi dans le groupe constitué par les composés de formules (1) ou (2),

$$[NR_4]^+ [B(CN)_4]^- \qquad (1),$$

$$[PR_4]^+ [B(CN)_4]^- \qquad (2),$$

où
R dans chaque cas, indépendamment l'un de l'autre, désigne

- alkyle à chaîne linéaire ou ramifiée ayant 1-20 atomes de C,
- alcényle à chaîne linéaire ou ramifiée ayant 2-20 atomes de C et une ou plusieurs doubles liaisons,
- alcynyle à chaîne linéaire ou ramifiée ayant 2-20 atomes de C et une ou plusieurs triples liaisons,
- cycloalkyle saturé, partiellement ou totalement insaturé ayant 5-7 atomes de C, pouvant être substitué par des groupements alkyle ayant 1-6 atomes de C,

à condition qu'au moins deux substituants R aient au moins 5 atomes de C, ou
est choisi dans le groupe constitué par les composés de formule (7),

$$[HétN]^+ [B(CN)_4]^- \qquad (7),$$

où

HétN⁺ désigne un cation hétérocyclique choisi dans le groupe constitué par

imidazolium

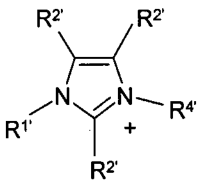

,

1H-pyrazolium

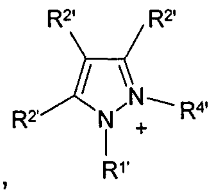

,

3H-pyrazolium

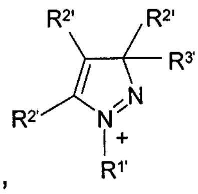

,

4H-pyrazolium

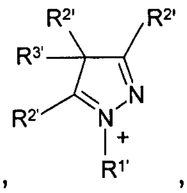

,

1-pyrazolinium

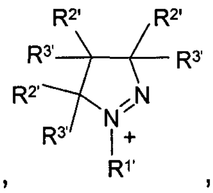

,

2-pyrazolinium

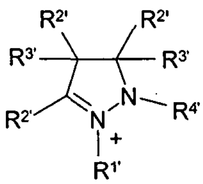

,

3-pyrazolinium

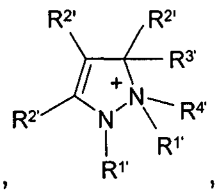

,

2,3-dihydroimidazolinium

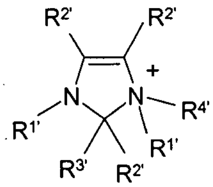

,

4,5-dihydroimidazolinium

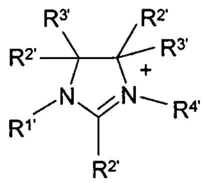

,

2,5-dihydroimidazolinium

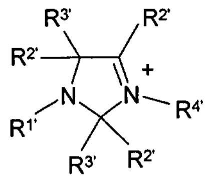

,

pyrrolidinium

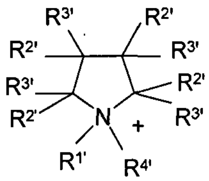

,

1,2,4-triazolium

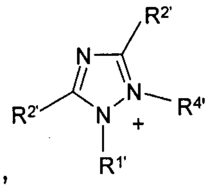

,

1,2,4-triazolium

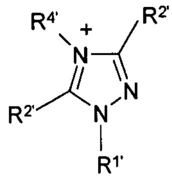

,

1,2,3-triazolium

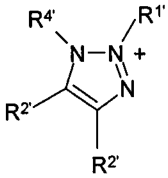

,

1,2,3-triazolium

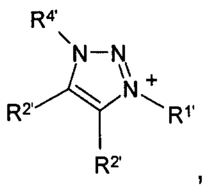

,

pyridinium

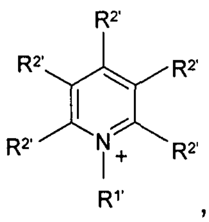

,

pyridazinium

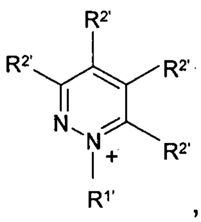

,

pyrimidinium

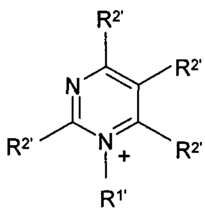

,

pipéridinium

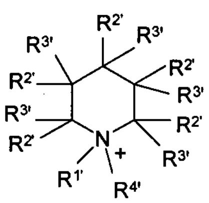

,

morpholinium

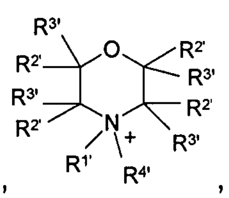

,

pipérazinium

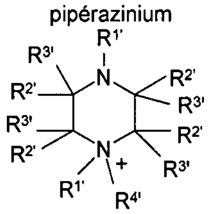

,

pipérazinium

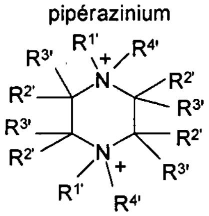

,

pyrazinium

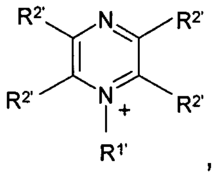

,

thiazolium

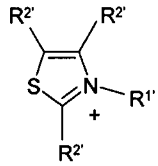

,

oxazolium

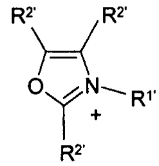

,

indolium

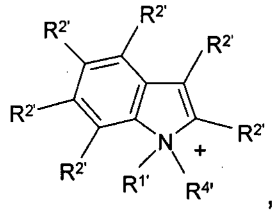

,,

quinoléinium

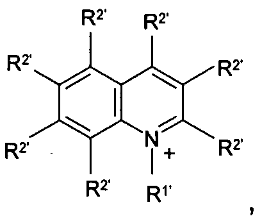

,

isoquinoléinium

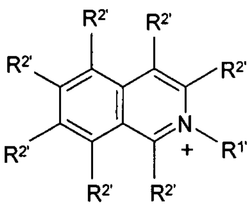

,

quinoxalinium

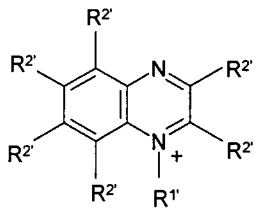

ou

indolinium

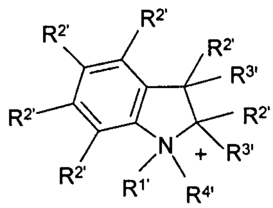

où les substituants

$R^{1'}$ à $R^{4'}$ désignent chacun, indépendamment les uns des autres,

- alkyle à chaîne linéaire ou ramifiée ayant 1-20 atomes de C,
- alcényle à chaîne linéaire ou ramifiée ayant 2-20 atomes de C et une ou plusieurs doubles liaisons,
- alcynyle à chaîne linéaire ou ramifiée ayant 2-20 atomes de C et une ou plusieurs triples liaisons,
- cycloalkyle saturé, partiellement ou totalement insaturé ayant 5-7 atomes de C, pouvant être substitué par des groupements alkyle ayant 1-6 atomes de C,

où les substituants $R^{1'}$, $R^{2'}$, $R^{3'}$ et/ou $R^{4'}$ peuvent également former ensemble un noyau et où le au moins un alcool est choisi dans le groupe constitué par l'éthanol, l'isopropanol, le propanol, le n-butanol ou les isomères de n-butanol, ou des mélanges de ceux-ci.

2. Méthode selon la revendication 1, **caractérisée en ce que** la méthode est effectuée de manière continue ou semi-continue.

3. Méthode selon la revendication 1, **caractérisée en ce que** le au moins un alcool est le n-butanol.

4. Méthode selon l'une ou plusieurs parmi les revendications 1 à 3, **caractérisée en ce que** la solution aqueuse comprenant au moins un alcool est un bouillon de fermentation.

5. Méthode selon l'une ou plusieurs parmi les revendications 1 à 4, **caractérisée en ce que** le bouillon de fermentation est issu d'une fermentation acétone-butanol-éthanol.

6. Méthode selon l'une ou plusieurs parmi les revendications 1 à 5, **caractérisée en ce que** le au moins un liquide ionique contenant des anions tétracyanoborate est choisi dans le groupe constitué par
le tétracyanoborate de 1-octyl-3-méthylimidazolium,
le tétracyanoborate de 1-décyl-3-méthylimidazolium,
le tétracyanoborate de 1-dodécyl-3-méthylimidazolium,
le tétracyanoborate de trihexyltétradécylammonium,
le tétracyanoborate de trihexyltétradécylphosphonium,
le tétracyanoborate de N-octylpyridinium,
le tétracyanoborate de 1-octyl-1-méthylpyrrolidinium,
le tétracyanoborate de N-octyl-N-méthylmorpholinium,
le tétracyanoborate de 1-octyl-1-méthylpipéridinium.

Fig. 1

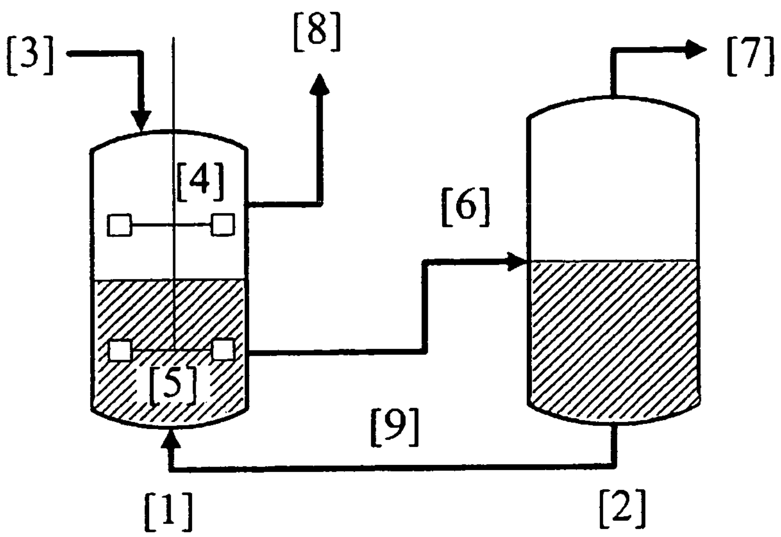

Fig. 2

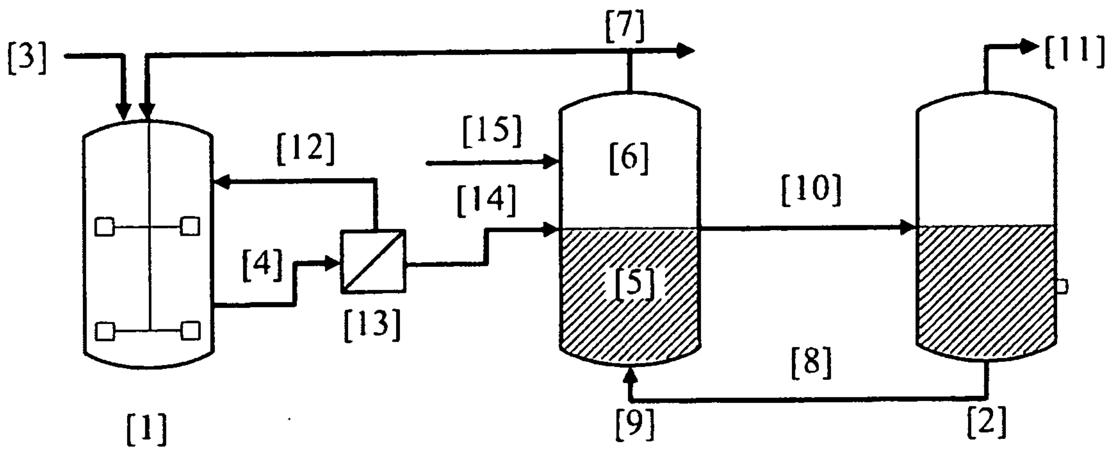

Fig. 3

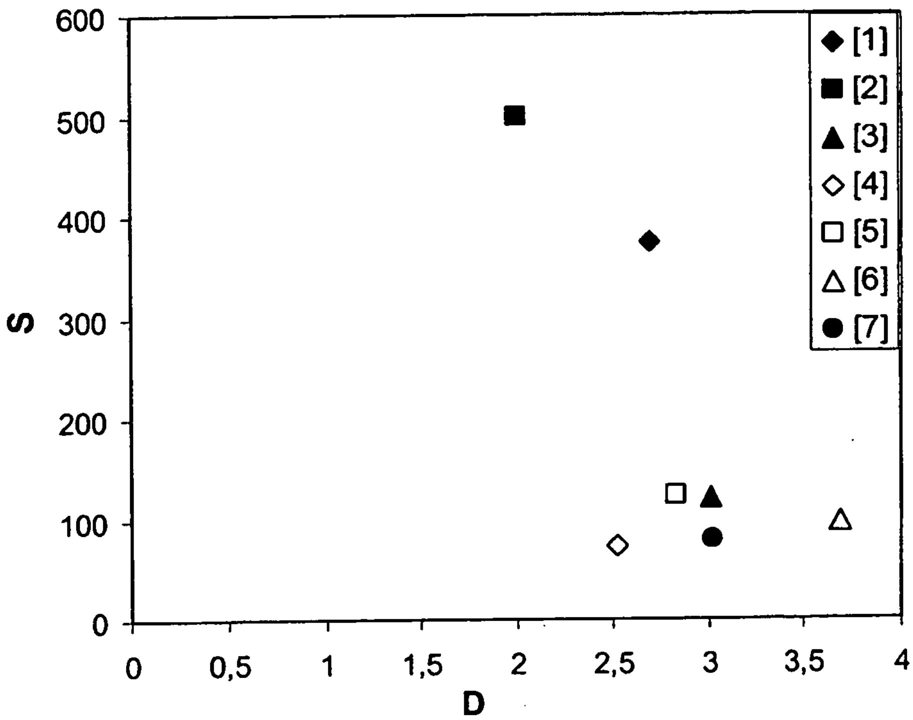

Fig. 4

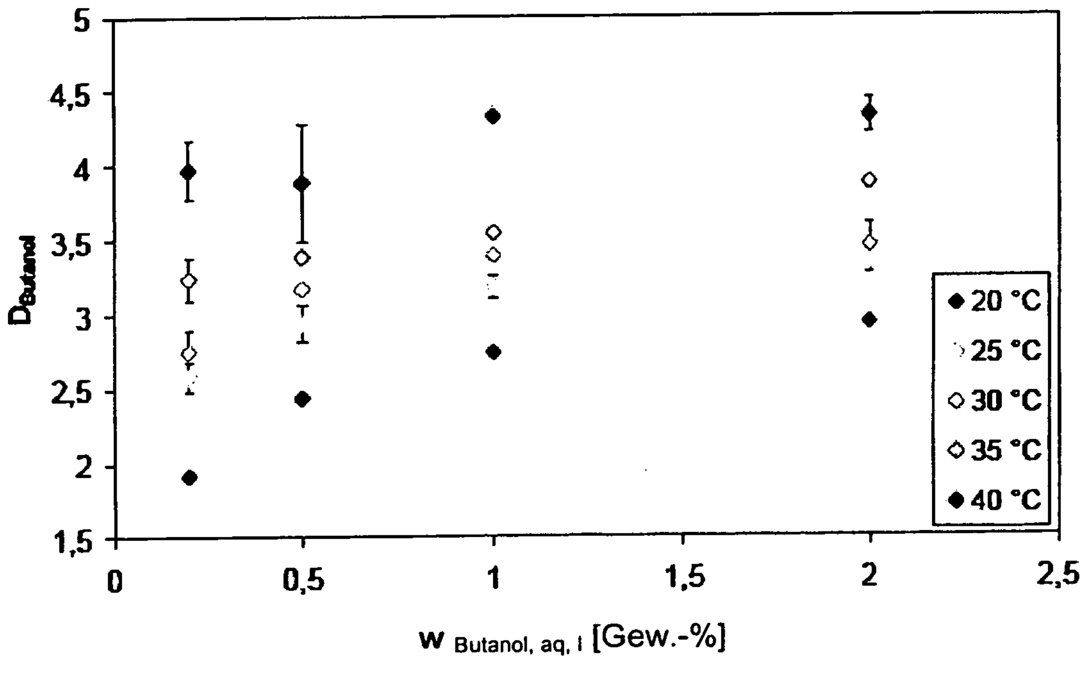
5
4,5
4
3,5
3
2,5
2
1,5
$D_{Butanol}$
0
0,5
1
1,5
2
2,5
◆ 20 °C
25 °C
◇ 30 °C
◇ 35 °C
◆ 40 °C
W Butanol, aq, l [Gew.-%]

Fig. 5

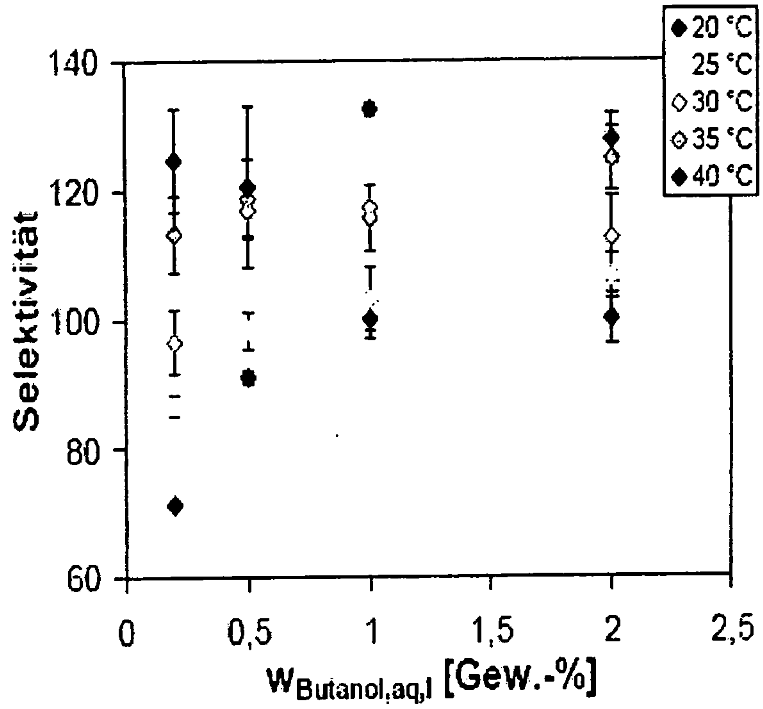

20 °C
25 °C
30 °C
35 °C
40 °C
140
120
100
80
60
Selektivität
0
0,5
1
1,5
2
2,5
$w_{Butanol,aq,l}$ [Gew.-%]

Fig. 6

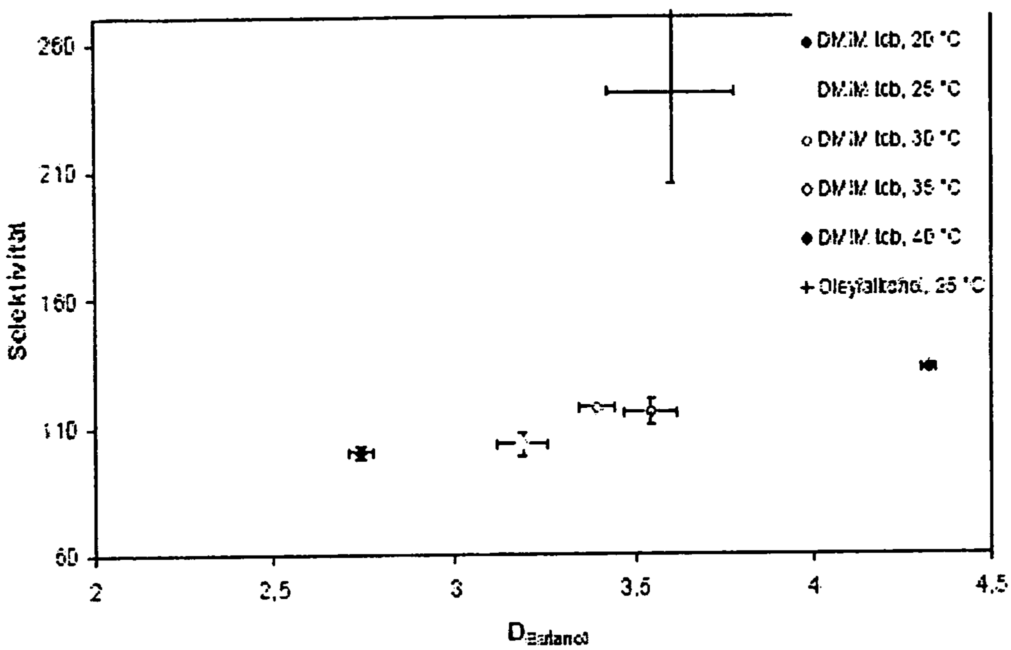
260
210
160
110
60
Selektivität
2
2,5
3
3,5
4
4,5
DMIM tcb, 20 °C
DMIM tcb, 25 °C
DMIM tcb, 30 °C
DMIM tcb, 35 °C
DMIM tcb, 40 °C
Oleylalkohol, 25 °C

Fig. 7

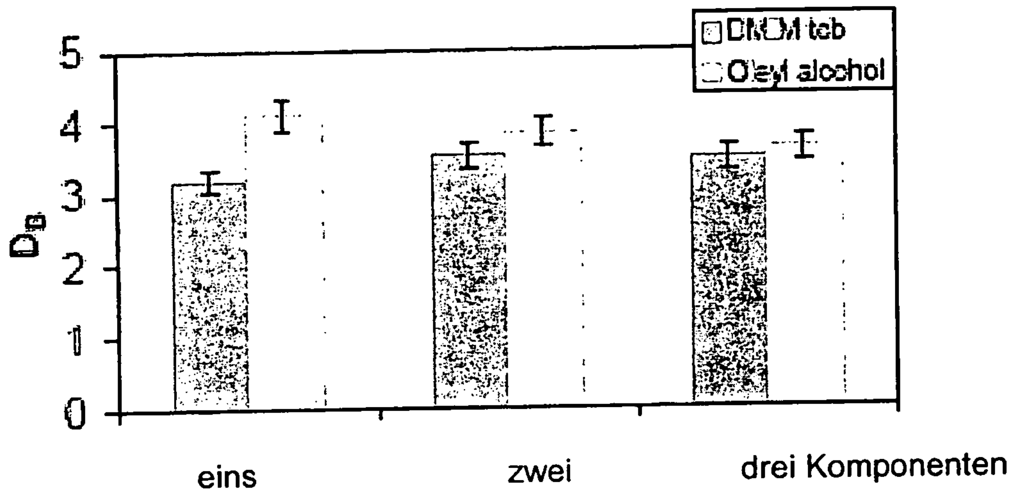
DMDM teb
Oleyl alcohol
$D_B$
0
1
2
3
4
5
eins
zwei
drei Komponenten

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 2004072089 A
- DE 102006024397


### In der Beschreibung aufgeführte Nicht-Patentliteratur


- **PAVEL IZÁK et al.** *Appl. Microbiol Biotechnol,* 2008, vol. 78, 597-602
- **A. CHAPEAUX et al.** *J. Chem. Eng. Data,* 2007, vol. 52, 2462-2467
- **J. RYDBERG ; M. COX ; C. MUSKAS ; G.R. CHOPPPIN.** *Solvent Extraction Principles and Practice,* 2004
- **R.H. PERRY.** Perry's chemical engineer's handbook. 1984
- **J. RYDBERG ; M. COX ; C. MUSKAS ; G.R. CHOPPPIN.** *Solvent Extraction Principles,* 2004
- **R. H. PERRY.** Perry's chemical engineer's handbook. 1984